# EUROPEAN PATENT APPLICATION

(11) **EP 4 101 845 A1**
(43) Date of publication of application: **14.12.2022**
(21) Application number: 21751481.9
(22) Date of filing: 03.02.2021
(51) Int. Cl.: C07D 233/90

(54) **METHOD FOR PRODUCING CARBOXYLATE COMPOUND AND METHOD FOR PRODUCING AMIDATE COMPOUND**

(30) Priority: 03.02.2020 JP 2020016172
(71) Applicant: Koei Chemical Company, Limited, Sodegaura-shi, Chiba 299-0266 (JP)
(72) Inventor: ONODA, Mitsuki, Sodegaura-shi, Chiba 299-0266 (JP); MIYAGI, Motoyoshi, Sodegaura-shi, Chiba 299-0266 (JP)
(74) Representative: Müller-Boré & Partner Patentanwälte PartG mbB
(86) International application number: PCT/JP2021/003818
(87) International publication number: WO 2021/157587

(57) **Abstract**

This invention provide a method for producing a carboxylate compound represented by the following formula (3a), the method comprising reacting an imidazolium carboxylic acid salt represented by the following formula (1) and a carbonic acid ester represented by the following formula (2): 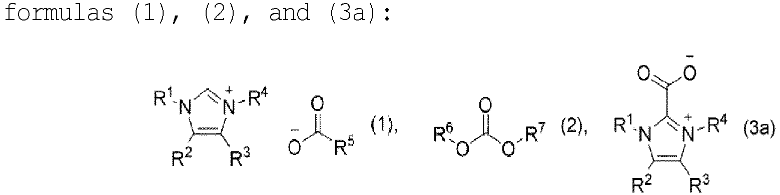 wherein R¹ to R⁷ are as defined in the specification.

## Description

### Technical Field

The present invention relates to a method for producing a carboxylate compound, and a method for producing an amidate compound.

### Background Art

Carboxylate compounds in which carbon dioxide is added to an imidazolium skeleton are known as stable N-heterocyclic carbene (hereinafter referred to as "NHC carbene") precursors and are used as catalysts for the production of urethane resins.

A known conventional method for producing carboxylate compounds is a method comprising reacting an NHC carbene with carbon dioxide (Non-patent Literature (NPL) 1).

It is also known that amidate compounds in which an isocyanate is added to the 2-position of an imidazolium skeleton can be used as catalysts for the production of urethane resins. A known method for producing amidate compounds is a method comprising reacting 1,3-dialkylimidazol-2-ylidene, which is an NHC carbene, with an isocyanate compound (NPL 2).

### Citation List

### Non-patent Literature

NPL 1: Journal of Organic Chemistry, 2009, vol. 74, pp. 7935-7942
NPL 2: Struct. Chem., 2013, vol. 24, pp. 2059-2068

### Summary of Invention

### Technical Problem

The methods described in NPL 1 and NPL 2, which comprise reacting an NHC carbene with carbon dioxide or an isocyanate, require production under water- and oxygen-free conditions using special equipment such as a glove box because NHC carbenes are generally unstable to oxygen or water. Accordingly, the methods described in NPL 1 and NPL 2 are not satisfactory from a practical standpoint.

The present invention was made in light of the above background art. An object of the present invention is to provide a method for producing a carboxylate compound or an amidate compound that does not require production under water- and oxygen-free conditions using special equipment such as a glove box.

### Solution to Problem

The present inventors conducted extensive research to solve the above problem, and found that a carboxylate compound can be produced by reacting an imidazolium carboxylic acid salt with a carbonic acid ester, such as a dialkyl carbonate or an alkylene carbonate. The present inventors also found that an amidate compound can be produced by reacting an imidazolium carboxylic acid salt with a carbonic acid ester, such as a dialkyl carbonate or an alkylene carbonate; and further reacting the resulting product with a nitrogen-containing compound, such as phenyl isocyanate. The present invention was thus accomplished.

Specifically, the present invention includes the following [1] to [9].
[1] A method for producing a carboxylate compound represented by the following formula (3a), the method comprising reacting an imidazolium carboxylic acid salt represented by the following formula (1) and a carbonic acid ester represented by the following formula (2): 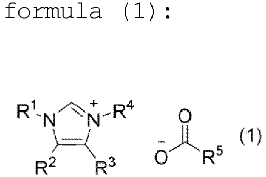 wherein R¹ and R⁴ are the same or different, and are each a C₁-C₂₀ hydrocarbon group optionally substituted with one or more heteroatoms; R² and R³ are the same or different, and are each a hydrogen atom or a C₁-C₂₀ hydrocarbon group optionally substituted with one or more heteroatoms; R² and R³, together with the carbon atoms to which they are attached, may form a ring structure; and R⁵ is a hydrogen atom or a C₁-C₂₀ hydrocarbon group optionally substituted with one or more heteroatoms; 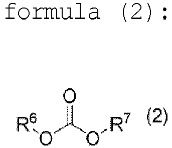 wherein R⁶ and R⁷ are the same or different, and are each a C₁-C₆ hydrocarbon group; and R⁶ and R⁷, together with the oxygen atoms to which they are attached, may form a ring structure; 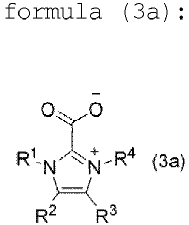 wherein R¹, R², R³, and R⁴ are as defined above.
[2] The method for producing a carboxylate compound according to [1], wherein the carbonic acid ester represented by formula (2) is dimethyl carbonate.
[3] The method for producing a carboxylate compound according to [1] or [2], wherein R² and R³ are hydrogen atoms.
[4] A method for producing an amidate compound represented by the following formula (5), the method comprising the following steps 1 and 2:
   step 1 of reacting an imidazolium carboxylic acid salt represented by formula (1) and a carbonic acid ester represented by formula (2) to obtain a reaction product (A): 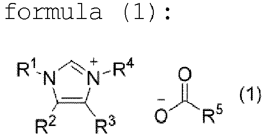 wherein R¹ and R⁴ are the same or different, and are each a C₁-C₂₀ hydrocarbon group optionally substituted with one or more heteroatoms; R² and R³ are the same or different, and are each a hydrogen atom or a C₁-C₂₀ hydrocarbon group optionally substituted with one or more heteroatoms; R² and R³, together with the carbon atoms to which they are attached, may form a ring structure; and R⁵ is a hydrogen atom or a C₁-C₂₀ hydrocarbon group optionally substituted with one or more heteroatoms; 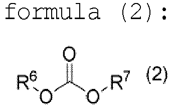 wherein R⁶ and R⁷ are the same or different, and are each a C₁-C₆ hydrocarbon group; and R⁶ and R⁷, together with the oxygen atoms to which they are attached, may form a ring structure; and
   step 2 of optionally heating the reaction product (A) obtained in step 1, and reacting the reaction product (A) with a nitrogen-containing compound represented by formula (4) optionally under heating, to obtain an amidate compound represented by formula (5): 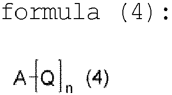 wherein A is a substituted or unsubstituted hydrocarbon group; Q is an -NCO group or -NHCO₂R⁸; R⁸ is a hydrocarbon group optionally substituted with one or more heteroatoms; and n is an integer of 1 or more; 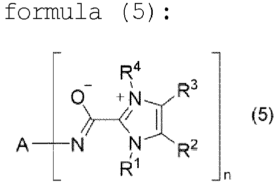 wherein A, R¹, R², R³, R⁴, and n are as defined above.
[5] The method for producing an amidate compound according to [4], wherein the reaction product (A) comprises a carboxylate compound represented by formula (3a): 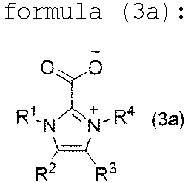 wherein R¹, R², R³, and R⁴ are as defined above.
[6] The method for producing an amidate compound according to [4] or [5], wherein the nitrogen-containing compound represented by formula (4) is a nitrogen-containing compound represented by any of the following formulas (4-1) to (4-3): formula (4-1):

   **R⁹-Q** (4-1)

   wherein Q is as defined above; and R⁹ is a hydrocarbon group optionally substituted with one or more halogen atoms or a hydrocarbon group optionally substituted with one or more heteroatoms; formula (4-2):

   **Q-R¹⁰-Q** **(4-2)**

   wherein each Q is the same or different and is as defined above; R¹⁰ is a divalent hydrocarbon group optionally substituted with one or more halogen atoms or a divalent hydrocarbon group optionally substituted with one or more heteroatoms; 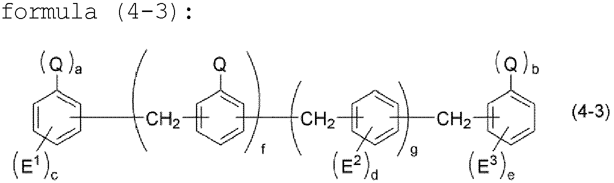 wherein each Q is the same or different and is as defined above; E¹, E², and E³ are each independently a hydrocarbon group optionally substituted with one or more halogen atoms, a hydrocarbon group optionally substituted with one or more heteroatoms, a halogen atom, a dialkylamino group, an alkoxy group, an aryloxy group, a nitro group, a cyano group, a sulfonyl group, an (alkylamino)carbonylamino group, a (dialkylamino)carbonylamino group, or an isocyanate group; f and g are each independently an integer of 0 to 4; a and b are each 0 or 1; and c, d, and e are each independently an integer of 0 to 4; provided that when f is 0, at least one of a or b is 1.
[7] The method for producing an amidate compound according to any one of [4] to [6], wherein the carbonic acid ester represented by formula (2) is dimethyl carbonate.
[8] The method for producing an amidate compound according to any one of [4] to [7], wherein R² and R³ are hydrogen atoms.
[9] The method for producing an amidate compound according to any one of [6] to [8], wherein R⁹ is an aromatic hydrocarbon group optionally substituted with one or more halogen atoms or an aromatic hydrocarbon group optionally substituted with one or more heteroatoms; and R¹⁰ is a divalent aromatic hydrocarbon group optionally substituted with one or more halogen atoms or a divalent aromatic hydrocarbon group optionally substituted with one or more heteroatoms.

### Advantageous Effects of Invention

The present invention can provide a method for producing a carboxylate compound and a method for producing an amidate compound, both of which do not require special equipment such as a glove box.

### Description of Embodiments

Embodiments of the present invention are described in detail below.

In the present invention, a carboxylate compound represented by formula (3a) (hereinafter referred to as "the carboxylate compound (3a)") is produced by reacting an imidazolium carboxylic acid salt represented by formula (1) (hereinafter referred to as "the imidazolium carboxylic acid salt (1)") and a carbonic acid ester represented by formula (2) (hereinafter referred to as "the carbonic acid ester (2)").

Moreover, in a method for producing an amidate compound represented by formula (5), the amidate compound is produced by the following step 1 and step 2.
Step 1: reacting an imidazolium carboxylic acid salt represented by formula (1) and a carbonic acid ester represented by formula (2) to obtain a reaction product (hereinafter referred to as "the reaction product (A)"): 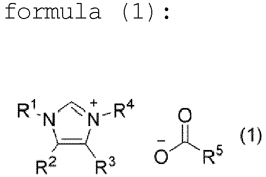 wherein R¹ and R⁴ are the same or different, and are each a C₁-C₂₀ hydrocarbon group optionally substituted with one or more heteroatoms; R² and R³ are the same or different, and are each a hydrogen atom or a C₁-C₂₀ hydrocarbon group optionally substituted with one or more heteroatoms; R² and R³, together with the carbon atoms to which they are attached, may form a ring structure; and R⁵ is a hydrogen atom or a C₁-C₂₀ hydrocarbon group optionally substituted with one or more heteroatoms; 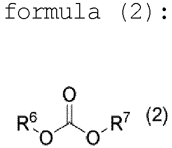 wherein R⁶ and R⁷ are the same or different, and are each a C₁-C₆ hydrocarbon group; and R⁶ and R⁷, together with the oxygen atoms to which they are attached, may form a ring structure.
Step 2: optionally heating the reaction product (A) obtained in step 1, and reacting the reaction product (A) with a nitrogen-containing compound represented by formula (4) (hereinafter referred to as "the nitrogen-containing compound (4)") optionally under heating, to obtain an amidate compound represented by formula (5) (hereinafter referred to as "the amidate compound (5)"):

   A-[Q]ₙ (4)

   wherein A is a hydrocarbon group optionally substituted with one or more halogen atoms or a hydrocarbon group optionally substituted with one or more heteroatoms; Q is an -NCO group or -NHCO₂R⁸; R⁸ is a hydrocarbon group optionally substituted with one or more heteroatoms; and n is an integer of 1 or more; 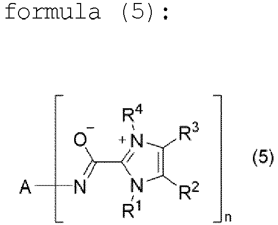 wherein A, R¹, R², R³, R⁴, and n are as defined above.

In one preferred embodiment of the present invention, the reaction product (A) comprises a carboxylate compound represented by formula (3a): 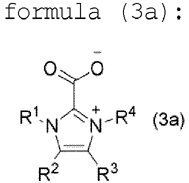 wherein R¹, R², R³, and R⁴ are as defined above.

In formula (1), R¹ and R⁴ are each a C₁-C₂₀ hydrocarbon group optionally substituted with one or more heteroatoms, preferably a C₁-C₁₂ hydrocarbon group optionally substituted with one or more heteroatoms, and particularly preferably a C₁-C₈ hydrocarbon group optionally substituted with one or more heteroatoms. In another embodiment, the C₁-C₂₀ hydrocarbon group optionally substituted with one or more heteroatoms is preferably a C₁-C₂₀ primary or secondary alkyl group optionally substituted with one or more heteroatoms, more preferably a C₁-C₁₂ primary or secondary alkyl group optionally substituted with one or more heteroatoms, and even more preferably a C₁-C₈ primary or secondary alkyl group optionally substituted with one or more heteroatoms.

Examples of the C₁-C₂₀ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, an octyl group, a 1,1,3,3-tetramethylbutyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, 2-(dimethylamino)ethyl group, and the like. Preferred are a methyl group, an ethyl group, a propyl group, an isopropyl group, a butyl group, an octyl group, a 1,1,3,3-tetramethylbutyl group, a dodecyl group, a cyclopentyl group, a cyclohexyl group, a 2-ethylhexyl group, a benzyl group, a phenyl group, and 2,4,6-trimethylphenyl group; and particularly preferred are a methyl group, an ethyl group, a butyl group, an octyl group, a 2-ethylhexyl group, a 1,1,3,3-tetramethylbutyl group, and a benzyl group.

Examples of heteroatoms in R¹ and R⁴ include nitrogen, oxygen, sulfur, and the like. When the hydrocarbon group is substituted with a heteroatom, such as oxygen, nitrogen, or sulfur, the hydrocarbon group has a group, such as -O-, -N<, -S-, or -SO₂-, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group.

R² and R³ are each a hydrogen atom or a C₁-C₂₀ hydrocarbon group optionally substituted with one or more heteroatoms, and preferably a hydrogen atom. The C₁-C₂₀ hydrocarbon group optionally substituted with one or more heteroatoms is preferably a C₁-C₆ hydrocarbon group optionally substituted with one or more heteroatoms, and particularly preferably a C₁-C₄ hydrocarbon group optionally substituted with one or more heteroatoms. Examples of the C₁-C₂₀ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, an octyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, a 2-(dimethylamino)ethyl group, and the like. Preferred are a methyl group, an ethyl group, a propyl group, an isopropyl group, a butyl group, a cyclopentyl group, a cyclohexyl group, a phenyl group, a 2-methoxyethyl group, a 2-ethoxyethyl group, and a 2-(dimethylamino)ethyl group; and particularly preferred are a methyl group, an ethyl group, a butyl group, a 2-methoxyethyl group, a 2-ethoxyethyl group, and a 2-(dimethylamino)ethyl group.

Examples of heteroatoms in R² and R³ include nitrogen, oxygen, sulfur, and the like. When the hydrocarbon group is substituted with a heteroatom, such as oxygen, nitrogen, or sulfur, the hydrocarbon group has a group, such as -O-, -N<, -S-, or -SO₂-, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group.

R² and R³, together with the carbon atoms to which they are attached, may form a ring structure. When R² and R³, together with the carbon atoms to which they are attached, form a ring structure, for example, a benzimidazolium ring structure as shown below can be formed: 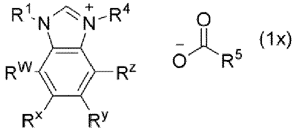 wherein R¹, R⁴, and R⁵ are as defined above; and R^{w}, R^{x}, R^{y}, and R^{z} are each a hydrogen atom or a C₁-C₂₀ hydrocarbon group.

R⁵ is a hydrogen atom or a C₁-C₂₀ hydrocarbon group optionally substituted with one or more heteroatoms, and preferably a C₁-C₂₀ hydrocarbon group optionally substituted with one or more heteroatoms. The C₁-C₂₀ hydrocarbon group optionally substituted with one or more heteroatoms is preferably a C₁-C₈ hydrocarbon group optionally substituted with one or more heteroatoms, and particularly preferably a C₁ or C₂ hydrocarbon group optionally substituted with one or more heteroatoms. Examples of the C₁-C₂₀ hydrocarbon group optionally substituted with one or more heteroatoms include a methyl group, an ethyl group, a propyl group, an isopropyl group, a butyl group, a sec-butyl group, a tert-butyl group, a pentyl group, a hexyl group, an octyl group, a 2-ethylhexyl group, a decyl group, a dodecyl group, a tetradecyl group, a hexadecyl group, an octadecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, a 2-(dimethylamino)ethyl group, and the like. Preferred are a methyl group, an ethyl group, a propyl group, an isopropyl group, a butyl group, a cyclopentyl group, a cyclohexyl group, a 2-ethylhexyl group, and a phenyl group; and particularly preferred are a methyl group and an ethyl group.

Examples of heteroatoms in R⁵ include nitrogen, oxygen, sulfur, and the like. When the hydrocarbon group is substituted with a heteroatom, such as oxygen, nitrogen, or sulfur, the hydrocarbon group has a group, such as -O-, -N<, -NH-, -S-, or -SO₂-, and the hydrocarbon chain is interrupted by such a group. When the hydrocarbon group is substituted with a heteroatom, such as oxygen, nitrogen, or sulfur, it is preferred that the hydrocarbon group is substituted with oxygen and that the hydrocarbon chain is interrupted by an -O- group. In another embodiment, when the hydrocarbon group is substituted with a heteroatom, such as oxygen, nitrogen, or sulfur, a hydrocarbon group having a group, such as -OH or -NH₂, may be formed.

Examples of the imidazolium carboxylic acid salt (1) include 1,3-dimethylimidazolium formate, 1-ethyl-3-methylimidazolium formate, 1-butyl-3-methylimidazolium formate, 1-methyl-3-octylimidazolium formate, 1-methyl-3-(1,1,3,3-tetramethylbutyl)imidazolium formate, 1-methyl-3-(2-ethylhexyl)imidazolium formate, 1-dodecyl-3-methylimidazolium formate, 1-methyl-3-octadecylimidazolium formate, 1-benzyl-3-methylimidazolium formate, 1,3-dibutylimidazolium formate, 1-butyl-3-ethylimidazolium formate, 1-butyl-3-octylimidazolium formate, 1-butyl-3-(1,1,3,3-tetramethylbutyl)imidazolium formate, 1-butyl-3-(2-ethylhexyl)imidazolium formate, 1-butyl-3-dodecylimidazolium formate, 1-butyl-3-octadecylimidazolium formate, 1-benzyl-3-butylimidazolium formate, 1,3-dioctylimidazolium formate, 1,3-bis(1,1,3,3-tetramethylbutyl)imidazolium formate, 1-ethyl-3-octylimidazolium formate, 1-ethyl-3-(1,1,3,3-tetramethylbutyl)imidazolium formate, 1-octyl-3-(2-ethylhexyl)imidazolium formate, 1-(1,1,3,3-tetramethylbutyl)-3-(2-ethylhexyl)imidazolium formate, 1-dodecyl-3-octylimidazolium formate, 1-dodecyl-3-(1,1,3,3-tetramethylbutyl)imidazolium formate, 1-octyl-3-octadecylimidazolium formate, 1-(1,1,3,3-tetramethylbutyl)-3-octadecylimidazolium formate, 1-benzyl-3-octylimidazolium formate, 1-benzyl-3-(1,1,3,3-tetramethylbutyl)imidazolium formate, 1,3-bis(2-ethylhexyl)imidazolium formate, 1-ethyl-3-(2-ethylhexyl)imidazolium formate, 1-(2-ethylhexyl)-3-dodecylimidazolium formate, 1-(2-ethylhexyl)-3-octadecylimidazolium formate, 1-benzyl-3-(2-ethylhexyl)imidazolium formate, 1,3-didodecylimidazolium formate, 1-dodecyl-3-octadecylimidazolium formate, 1-benzyl-3-dodecylimidazolium formate, 1,3-dioctadecylimidazolium formate, 1-benzyl-3-octadecylimidazolium formate, 1,3-dibenzylimidazolium formate;

1,3-dimethylimidazolium acetate, 1-ethyl-3-methylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1-methyl-3-octadecylimidazolium acetate, 1-benzyl-3-methylimidazolium acetate, 1,3-dibutylimidazolium acetate, 1-butyl-3-ethylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1-butyl-3-octadecylimidazolium acetate, 1-benzyl-3-butylimidazolium acetate, 1,3-dioctylimidazolium acetate, 1,3-bis(1,1,3,3-tetramethylbutyl)imidazolium acetate, 1-ethyl-3-octylimidazolium acetate, 1-ethyl-3-(1,1,3,3-tetramethylbutyl)imidazolium acetate, 1-octyl-3-(2-ethylhexyl)imidazolium acetate, 1-(1,1,3,3-tetramethylbutyl)-3-(2-ethylhexyl)imidazolium acetate, 1-dodecyl-3-octylimidazolium acetate, 1-dodecyl-3-(1,1,3,3-tetramethylbutyl)imidazolium acetate, 1-octyl-3-octadecylimidazolium acetate, 1-(1,1,3,3-tetramethylbutyl)-3-octadecylimidazolium acetate, 1-benzyl-3-octylimidazolium acetate, 1-benzyl-3-(1,1,3,3-tetramethylbutyl)imidazolium acetate, 1,3-bis(2-ethylhexyl)imidazolium acetate, 1-ethyl-3-(2-ethylhexyl)imidazolium acetate, 1-(2-ethylhexyl)-3-dodecylimidazolium acetate, 1-(2-ethylhexyl)-3-octadecylimidazolium acetate, 1-benzyl-3-(2-ethylhexyl)imidazolium acetate, 1,3-didodecylimidazolium acetate, 1-dodecyl-3-octadecylimidazolium acetate, 1-benzyl-3-dodecylimidazolium acetate, 1,3-dioctadecylimidazolium acetate, 1-benzyl-3-octadecylimidazolium acetate, 1,3-dibenzylimidazolium acetate;

1,3-dimethylimidazolium 2-ethylhexanoate, 1-ethyl-3-methylimidazolium 2-ethylhexanoate, 1-butyl-3-methylimidazolium 2-ethylhexanoate, 1-methyl-3-octylimidazolium 2-ethylhexanoate, 1-methyl-3-(1,1,3,3-tetramethylbutyl)imidazolium 2-ethylhexanoate, 1-methyl-3-(2-ethylhexyl)imidazolium 2-ethylhexanoate, 1-dodecyl-3-methylimidazolium 2-ethylhexanoate, 1-methyl-3-octadecylimidazolium 2-ethylhexanoate, 1-benzyl-3-methylimidazolium 2-ethylhexanoate, 1,3-dibutylimidazolium 2-ethylhexanoate, 1-butyl-3-ethylimidazolium 2-ethylhexanoate, 1-butyl-3-octylimidazolium 2-ethylhexanoate, 1-butyl-3-(1,1,3,3-tetramethylbutyl)imidazolium 2-ethylhexanoate, 1-butyl-3-(2-ethylhexyl)imidazolium 2-ethylhexanoate, 1-butyl-3-dodecylimidazolium 2-ethylhexanoate, 1-butyl-3-octadecylimidazolium 2-ethylhexanoate, 1-benzyl-3-butylimidazolium 2-ethylhexanoate, 1,3-dioctylimidazolium 2-ethylhexanoate, 1,3-bis(1,1,3,3-tetramethylbutyl)imidazolium 2-ethylhexanoate, 1-ethyl-3-octylimidazolium 2-ethylhexanoate, 1-ethyl-3-(1,1,3,3-tetramethylbutyl)imidazolium 2-ethylhexanoate, 1-octyl-3-(2-ethylhexyl)imidazolium 2-ethylhexanoate, 1-(1,1,3,3-tetramethylbutyl)-3-(2-ethylhexyl)imidazolium 2-ethylhexanoate, 1-dodecyl-3-octylimidazolium 2-ethylhexanoate, 1-dodecyl-3-(1,1,3,3-tetramethylbutyl)imidazolium 2-ethylhexanoate, 1-octyl-3-octadecylimidazolium 2-ethylhexanoate, 1-(1,1,3,3-tetramethylbutyl)-3-octadecylimidazolium 2-ethylhexanoate, 1-benzyl-3-octylimidazolium 2-ethylhexanoate, 1-benzyl-3-(1,1,3,3-tetramethylbutyl)imidazolium 2-ethylhexanoate, 1,3-bis(2-ethylhexyl)imidazolium 2-ethylhexanoate, 1-ethyl-3-(2-ethylhexyl)imidazolium 2-ethylhexanoate, 1-(2-ethylhexyl)-3-dodecylimidazolium 2-ethylhexanoate, 1-(2-ethylhexyl)-3-octadecylimidazolium 2-ethylhexanoate, 1-benzyl-3-(2-ethylhexyl)imidazolium 2-ethylhexanoate, 1,3-didodecylimidazolium 2-ethylhexanoate, 1-dodecyl-3-octadecylimidazolium 2-ethylhexanoate, 1-benzyl-3-dodecylimidazolium 2-ethylhexanoate, 1,3-dioctadecylimidazolium 2-ethylhexanoate, 1-benzyl-3-octadecylimidazolium 2-ethylhexanoate, 1,3-dibenzylimidazolium 2-ethylhexanoate; and

1,3-dimethylbenzimidazolium formate, 1,3-dimethylbenzimidazolium acetate, and 3-dimethylbenzimidazolium 2-ethylhexanoate.

The imidazolium carboxylic acid salt (1) is preferably 1,3-dimethylimidazolium acetate, 1-ethyl-3-methylimidazolium acetate, 1-butyl-3-methylimidazolium acetate, 1-methyl-3-octylimidazolium acetate, 1-methyl-3-(1,1,3,3-tetramethylbutyl)imidazolium acetate, 1-methyl-3-(2-ethylhexyl)imidazolium acetate, 1-dodecyl-3-methylimidazolium acetate, 1-benzyl-3-methylimidazolium acetate, 1,3-dibutylimidazolium acetate, 1-butyl-3-ethylimidazolium acetate, 1-butyl-3-octylimidazolium acetate, 1-butyl-3-(1,1,3,3-tetramethylbutyl)imidazolium acetate, 1-butyl-3-(2-ethylhexyl)imidazolium acetate, 1-butyl-3-dodecylimidazolium acetate, 1-benzyl-3-butylimidazolium acetate, 1,3-dioctylimidazolium acetate, 1,3-bis(1,1,3,3-tetramethylbutyl)imidazolium acetate, 1-ethyl-3-octylimidazolium acetate, 1-ethyl-3-(1,1,3,3-tetramethylbutyl)imidazolium acetate, 1-octyl-3-(2-ethylhexyl)imidazolium acetate, 1-(1,1,3,3-tetramethylbutyl)-3-(2-ethylhexyl)imidazolium acetate, 1-dodecyl-3-octylimidazolium acetate, 1-dodecyl-3-(1,1,3,3-tetramethylbutyl)imidazolium acetate, 1-benzyl-3-octylimidazolium acetate, 1-benzyl-3-(1,1,3,3-tetramethylbutyl)imidazolium acetate, 1,3-bis(2-ethylhexyl)imidazolium acetate, 1-ethyl-3-(2-ethylhexyl)imidazolium acetate, 1-(2-ethylhexyl)-3-dodecylimidazolium acetate, 1-benzyl-3-(2-ethylhexyl)imidazolium acetate, 1,3-didodecylimidazolium acetate, 1-benzyl-3-dodecylimidazolium acetate, or 1,3-dibenzylimidazolium acetate.

The imidazolium carboxylic acid salt (1) may be a commercial product. The imidazolium carboxylic acid salt (1) may be a salt obtained by a known method or a salt produced by a method explained below.

A dicarbonyl compound represented by the following formula (6), a primary amine compound represented by the following formula (7a), a primary amine compound represented by the following formula (7b), formaldehyde, and a carboxylic acid represented by the following formula (8) are reacted to obtain a carboxylate compound of formula (1). 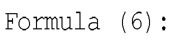 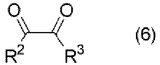 wherein R² and R³ are as defined above.

Formula (7a): R¹-NH₂ (7a)

wherein R¹ is as defined above.

Formula (7b): **R⁴-NH₂ (7b)**

wherein R⁴ is as defined above. 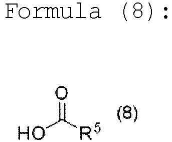 wherein R⁵ is as defined above.

Examples of the dicarbonyl compound represented by formula (6) (hereinafter referred to as "the dicarbonyl compound (6)") include glyoxal, diacetyl, 3,4-hexanedione, 2,3-pentanedione, 2,3-heptanedione, 5-methyl-2,3-hexanedione, 3-methyl-2,3-cyclopentanedione, 1,2-cyclohexanedione, 1-phenyl-1,2-propanedione, and dibenzoyl; preferably glyoxal and diacetyl; and more preferably glyoxal.

The primary amine compound represented by formula (7a) (hereinafter referred to as "the primary amine compound (7a)") and the primary amine compound represented by formula (7b) (hereinafter referred to as "the primary amine compound (7b)") are at least one primary amine compound selected from the group consisting of methylamine, ethylamine, propylamine, isopropylamine, butylamine, tert-butylamine, hexylamine, octylamine, 1,1,3,3-tetramethylbutylamine, 2-ethylhexylamine, dodecylamine, tetradecylamine, hexadecylamine, octadecylamine, 2-methoxyethylamine, 2-ethoxyethylamine, 3-methoxypropylamine, 3-ethoxypropylamine, 3-propoxypropylamine, 3-isopropoxypropylamine, 3-butoxypropylamine, 3-(2-ethylhexyloxy)propylamine, allylamine, benzylamine, aniline, 2,6-diisopropylaniline, and 2,4,6-trimethylaniline; preferably methylamine, ethylamine, butylamine, hexylamine, octylamine, (1,1,3,3-tetramethylbutyl)amine, 2-ethylhexylamine, dodecylamine, octadecylamine, and benzylamine; and more preferably butylamine, octylamine, 1,1,3,3-tetramethylbutylamine, 2-ethylhexylamine, and benzylamine.

Examples of the carboxylic acid represented by formula (8) (hereinafter referred to as "the carboxylic acid (8)") include carboxylic acids, such as formic acid, acetic acid, propionic acid, butyric acid, pentanoic acid, hexanoic acid, heptanoic acid, octanoic acid, 2-ethylhexanoic acid, capric acid, lauric acid, tetradecylic acid, palmitic acid, octadecylic acid, cyclohexanoic acid, ethoxyacetic acid, propoxyacetic acid, 2-(2-methoxyethoxy)acetic acid, 2-(2-ethoxyethoxy)acetic acid, 2-(2-propoxyethoxy)acetic acid, 3-methoxypropanoic acid, 3-ethoxypropanoic acid, 3-(2-methoxyethoxy)propanoic acid, 3-(2-ethoxyethoxy)propanoic acid, 3-(2-propoxyethoxy)propanoic acid, 3-(3-methoxypropoxy)propanoic acid, 3-(3-ethoxypropoxy)propanoic acid, 3-(3-propoxypropoxy)propanoic acid, oleic acid, linoleic acid, sorbic acid, benzoic acid, phthalic acid, isophthalic acid, terephthalic acid, lactic acid, salicylic acid, and trifluoroacetic acid. Preferred are formic acid, acetic acid, propionic acid, butyric acid, pentanoic acid, hexanoic acid, heptanoic acid, octanoic acid, and 2-ethylhexanoic acid, and more preferred is acetic acid.

As the dicarbonyl compound (6), an aqueous solution or an alcohol solution, such as methanol or butanol, may be used as is.

The amounts of the primary amine compounds (7a) and (7b) (the primary amine compounds (7a) and (7b) are hereinafter collectively referred to as "the amine compounds (7)") used are such that the total amount of the primary amine compounds (7a) and (7b) is generally 0.1 to 10 mol, and preferably 0.5 to 3 mol, per mol of the dicarbonyl compound (6). When the primary amine compounds (7) are allowed to react in an amount of 2 mol per mole of the dicarbonyl compound (6), 1 mol of the imidazolium carboxylic acid salt (1) is obtained. For example, when the primary amine compounds (7) are used in an amount of less than 2 mol, the imidazolium carboxylic acid salt (1) and a monocarbonylmonoamino compound obtained by reacting the dicarbonyl compound (6) (starting material) with 1 mol of the amine compounds (7) are obtained as a mixture. When the primary amine compounds (7) are used in an amount of more than 2 mol per mole of the dicarbonyl compound (6), an excess amount of the primary amine compounds (7) is present in addition to the desired imidazolium carboxylic acid salt (1). The carboxylate compound (3a) can be obtained even when the imidazolium carboxylic acid salt (1) present together with such a compound other than the imidazolium carboxylic acid salt (1) is used.

The ratio (molar ratio) of the primary amine compound (7a) to the primary amine compound (7b) is not particularly limited, and is such that primary amine compound (7a):primary amine compound (7b) = 0:100 to 100:0. When primary amine compound (7a):primary amine compound (7b) = 0:100 or primary amine compound (7a):primary amine compound (7b) = 100:0, R¹=R⁴. Even when primary amine compound (7a):primary amine compound (7b) = 1:1, the compound of formula (1) obtained is a mixture of a combination of three kinds of compounds (R¹, R¹), (R¹, R⁴), and (R⁴, R⁴). When primary amine compound (7a):primary amine compound (7b) = 0:100 to 100:0, the ratio of the combination of three kinds of compounds (R¹, R¹), (R¹, R⁴), and (R⁴, R⁴) varies. In these three kinds of compounds, R¹ and R⁴ may be the same or different, and these compounds are included in the compound of formula (1).

As the formaldehyde, an aqueous solution or an alcohol solution, such as methanol or butanol, may be used as is. The amount of formaldehyde used is generally 0.1 to 10 mol, and preferably 0.5 to 5.0 mol, per mol of the dicarbonyl compound (6) .

The amount of the carboxylic acid (8) used is generally 0.1 to 10 mol, preferably 0.5 to 2 mol, and more preferably 1 to 1.5 mol, per mol of the dicarbonyl compound (6).

The optimal reaction temperature varies depending on the starting materials, solvents, etc. used, but is generally -10°C or higher, and preferably 0°C to 100°C. The reaction time is generally 0.1 to 24 hours, and preferably 1 to 10 hours.

A solvent may or may not be used. When a solvent is used, the solvent used is not particularly limited, as long as it does not affect the reaction. Specific examples of solvents include aromatic hydrocarbon solvents, such as toluene, benzene, and xylene; hydrocarbon solvents, such as methylcyclohexane, cyclohexane, hexane, heptane, and octane; halogenated hydrocarbon solvents, such as dichloromethane and chloroform; ether solvents, such as diethyl ether, tetrahydrofuran, and 1,4-dioxane; alcohol solvents, such as methanol and ethanol; N,N-dimethylformamide, acetonitrile, water, and the like. Preferred are aromatic hydrocarbon solvents, alcohol solvents, and water; and particularly preferred are toluene and water. The solvents can be used as a mixture of two or more, if necessary.

The amount of solvent used is generally 50 parts by mass or less, and preferably 0.1 to 10 parts by mass, per part by mass of the dicarbonyl compound (6).

The reaction may be performed, if necessary, in an inert gas atmosphere, such as nitrogen, argon, or helium, which do not affect the reaction.

After completion of the reaction, the imidazolium carboxylic acid salt (1) can be isolated, for example, by removing impurities (e.g., unreacted starting materials) by washing with an organic solvent, or concentrating the reaction liquid, and may be purified by recrystallization etc., if necessary.

The carbonic acid ester represented by formula (2) (hereinafter referred to as "the carbonic acid ester (2)") is described below.

In formula (2), R⁶ and R⁷ are the same or different, and are each a C₁-C₆ hydrocarbon group, preferably a C₁-C₄ hydrocarbon group, and particularly preferably a methyl group. In another embodiment, the C₁-C₆ hydrocarbon group is preferably a C₁-C₆ alkyl group, and more preferably C₁-C₄ alkyl group. R⁶ and R⁷, together with the oxygen atoms to which they are attached, may form a ring structure.

Specific examples of the carbonic acid ester (2) include dialkyl carbonates, such as dimethyl carbonate, diethyl carbonate, dipropyl carbonate, dibutyl carbonate, dipentyl carbonate, and dihexyl carbonate; and alkylene carbonates, such as ethylene carbonate, propylene carbonate, and butylene carbonate. Preferred are dimethyl carbonate, diethyl carbonate, dipropyl carbonate, and dibutyl carbonate; and particularly preferred is dimethyl carbonate.

In the production of the carboxylate compound (3a) and step 1, the amount of the carbonic acid ester (2) used is generally 1 mol or more, and preferably 1 to 6 mol, per mol of the imidazolium carboxylic acid salt (1). When excess carboxylic acid and water are contained in the imidazolium carboxylic acid salt (1), they react with the carbonic acid ester (2); thus, it is preferable to use the carbonic acid ester (2) in an amount of generally 1 mol or more, and preferably an excess of 1 to 6 mol, per mol of the total of excess carboxylic acid and water in the imidazolium carboxylic acid salt (1).

In the production of the carboxylate compound (3a) and step 1, a solvent may or may not be used. When a solvent is used, the solvent used is not particularly limited, as long as it does not affect the reaction. Specific examples of solvents include monovalent alcohol solvents, such as methanol, ethanol, propanol, butanol, pentanol, hexanol, 1-methoxy-2-propanol, and ethoxyethanol; polyol solvents, such as ethylene glycol, propylene glycol, and diethylene glycol; glycol monoalkyl ether solvents, such as dipropylene glycol monobutyl ether, dipropylene glycol monomethyl ether, and tripropylene glycol monomethyl ether; aromatic hydrocarbon solvents, such as toluene, benzene, and xylene; aliphatic hydrocarbon solvents, such as methylcyclohexane, cyclohexane, hexane, heptane, and octane; ester solvents, such as ethyl acetate and butyl acetate; ketone solvents, such as methyl ethyl ketone and 4-methyl-2-pentanone; and the like. Preferred are monovalent alcohol solvents; and particularly preferred is methanol. The amount of solvent used is generally 50 parts by mass or less, and preferably 10 parts by mass or less, per part by mass of the imidazolium carboxylic acid salt (1).

In the production of the carboxylate compound (3a) and step 1, the optimal reaction temperature varies depending on the starting materials, solvents, etc. used, but is generally room temperature or higher, and preferably 20 to 200°C. The reaction time is generally 0.1 to 48 hours, and preferably 1 to 24 hours. In the present specification, room temperature means about 20°C.

In the production of the carboxylate compound (3a) and step 1, the reaction may be performed, if necessary, in an inert gas atmosphere, such as nitrogen, argon, or helium, which do not affect the reaction.

In the production of the carboxylate compound (3a), after completion of the reaction, the carboxylate compound (3a) can be obtained by concentrating the reaction liquid, and removing the solvent, and may be purified by recrystallization, column separation, etc., if necessary.

Further, the carboxylate compound (3a) can be reacted with a nitrogen-containing compound represented by formula (4) optionally under heating, to obtain an amidate compound represented by formula (5). When the amidate compound represented by formula (5) is produced by this method, the same method as step 2 described below can be used.

The reaction product (A) obtained in step 1 may be used directly as the reaction product (A) used as a starting material in step 2. If the carbonic acid ester (2) or solvent remains in the obtained reaction product (A) in step 1, the reaction liquid can be concentrated to remove the carbonic acid ester (2) or solvent, and the resulting reaction product (A) can be used as the reaction product (A) used as a starting material in step 2.

### Step 2

An amidate compound represented by formula (5) can be produced by optionally heating the reaction product (A) obtained in step 1, and reacting the reaction product (A) with a nitrogen-containing compound represented by formula (4) optionally under heating. The phrase "under heating" means 40°C to the boiling point of the solvent, for example 40 to 200°C. The reaction of step 2 may be carried out at room temperature or under heating.

As mentioned above, in one preferred embodiment of the present invention, the reaction product (A) contains the carboxylate compound (3a), and the reaction product (A) can contain, in addition to the carboxylate compound (3a), compounds represented by the following formulas (3b) to (3f) (hereinafter referred to as "the compound (3b)," "the compound (3c)," "the compound (3d)," "the compound (3e)," and "the compound (3f)," respectively) (reaction scheme 1). However, the compound (3b) can be contained in the reaction product (A) when R² is a hydrogen atom in formula (3a), and the compound (3c) can be contained in the reaction product (A) when R³ is a hydrogen atom in formula (3a) . 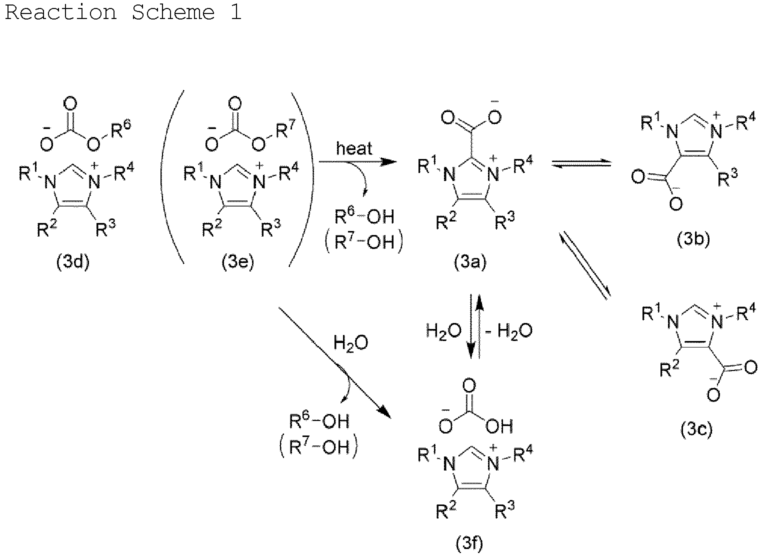 wherein R¹ to R⁴, R⁶, and R⁷ are as defined above.

As an intermediate in the reaction of step 1, the compound represented by formula (3d) and/or the compound represented by formula (3e) is produced; and from these compounds, the carboxylate compound (3a) and/or the compound represented by formula (3f) is produced. The compound (3b) can be produced in equilibrium with the carboxylate compound (3a) when R² is a hydrogen atom in formula (3a), and the compound represented by formula (3c) can be produced in equilibrium with the carboxylate compound (3a) when R³ is a hydrogen atom.

The compounds (3b) and (3c) are in equilibrium with the carboxylate compound (3a). Thus, consumption of the carboxylate compound (3a) in the step 2 shifts the equilibrium toward the production of compound (3a) from compound (3b) and compound (3b). It is thus inferred that the compounds (3b) and (3c) can be treated as being equivalent to the carboxylate compound (3a).

As shown in the following reaction scheme 2, the compounds (3d) and (3e) can be reacted with a compound represented by formula (4a) in the same manner as the carboxylate compound (3a) to produce the amidate compound (5). Thus, the compounds (3d) and (3e) can be treated as being equivalent to the carboxylate compound (3a). In addition, a compound represented by formula (4b') or (4b") in the reaction scheme below is produced as a by-product. The compound represented by formula (4b') or (4b") is decomposed by heat into the corresponding alcohol compound represented by formula (9a) or (9b) and an isocyanate compound represented by formula (4a), as shown in the reaction scheme below. The isocyanate compound represented by formula (4a) produced by the decomposition is consumed in a reaction with the carboxylate compound (3a) and the compounds (3b) to (3f) contained in the reaction product (A). In the following reaction scheme 2, the case in which Q is an -NCO group in formula (4) is shown; however, the same also applies to the case in which Q is -NHCO₂R⁸. 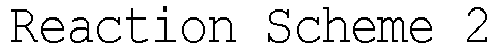 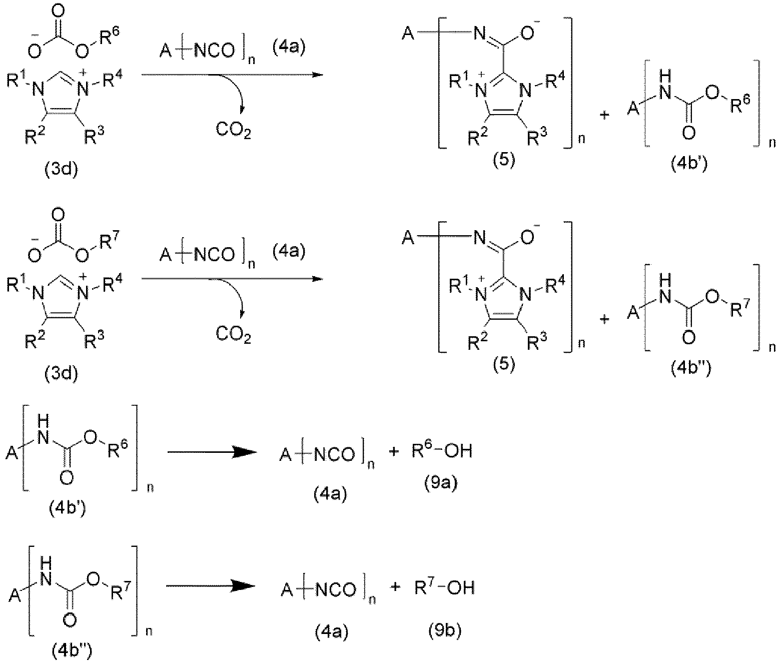 wherein R¹ to R⁴, R⁶ to R⁷, A, and n are as defined above.

In the reaction product (A), the compound (3f) can be transformed into the carboxylate compound (3a) by dehydration. The dehydration reaction is considered to be proceeded easily occur at ordinary temperature and ordinary pressure. Thus, it is inferred that the compound (3f) can be treated as being equivalent to the carboxylate compound (3a).

The nitrogen-containing compound represented by formula (4) (hereinafter referred to as "the nitrogen-containing compound (4)") is described below.

In formula (4), A is a substituted or unsubstituted hydrocarbon group, preferably a substituted or unsubstituted C₁-C₁₀₀ hydrocarbon group, more preferably a hydrocarbon group optionally substituted with one or more halogen atoms or a hydrocarbon group optionally substituted with one or more heteroatoms, and particularly preferably a C₁-C₃₀ hydrocarbon group optionally substituted with one or more halogen atoms or a C₁-C₃₀ hydrocarbon group optionally substituted with one or more heteroatoms. Examples of the "hydrocarbon group" of the substituted or unsubstituted hydrocarbon group represented by A include aryl groups, alkyl groups, and arylalkyl groups. Specific examples include linear, branched, or cyclic alkyl groups, such as methyl, ethyl, n-propyl, isopropyl, n-butyl, n-pentyl, n-hexyl, n-heptyl, n-octyl, n-nonyl, n-decyl, n-dodecyl, n-octadecyl, and cyclohexyl; aryl groups, such as phenyl, 2-methylphenyl, 2,6-dimethylphenyl, 2,4-dimethylphenyl, 2,3-dimethylphenyl, and naphthyl; arylalkyl groups, such as phenylmethyl, phenylethyl, 1-phenylpropyl, 2-phenylpropyl, 1-phenylbutyl, 2-phenylbutyl, naphthylmethyl, and naphthylethyl; arylalkyl groups obtained by suitably combining the above alkyl groups and aryl groups; and the like.

When A is a substituted hydrocarbon group, examples of substituents include halogen atoms, such as fluorine, chlorine, bromine, and iodine; dialkylamino groups, such as dimethylamino; alkoxy groups, such as methoxy and ethoxy; aryloxy groups, such as benzyloxy; halogenated alkyl groups, such as trifluoromethyl; nitro groups, cyano groups, sulfonyl groups, (alkylamino)carbonylamino groups, (dialkylamino)carbonylamino groups, isocyanate groups, and the like. Moreover, the hydrocarbon group A may be substituted with one or more heteroatoms, such as oxygen, nitrogen, and sulfur. When the hydrocarbon group A is substituted with a heteroatom, such as oxygen, nitrogen, or sulfur, the hydrocarbon group has a group, such as -O-, -N<, -S-, or -SO₂-, and the hydrocarbon chain is interrupted by such a group.

Examples of the alkyl moiety of the above dialkylamino groups, alkoxy groups, halogenated alkyl groups, (alkylamino)carbonylamino groups, and (dialkylamino)carbonylamino groups include linear or branched C₁-C₆ alkyl groups, such as methyl, ethyl, n-propyl, isopropyl, n-butyl, isobutyl, sec-butyl, tert-butyl, and n-pentyl. The number of carbon atoms in the alkyl group is preferably 1 to 3, and more preferably 1 or 2.

Examples of the aryl moiety of the above aryloxy groups include C₆-C₁₀ aryl groups. Specific examples include a phenyl group, a naphthyl group, and the like.

The number of substituents is 1 to 5, preferably 1 to 3, and more preferably 1 or 2.

In formula (4), n is an integer of 1 or more, preferably 1 to 6, more preferably 1 to 4, and particularly preferably 1 or 2.

In formula (4), Q is an -NCO group or an -NHCO₂R^{B} group. R⁸ is a hydrocarbon group that may contain one or more heteroatoms, preferably a C₁-C₅₀ hydrocarbon group that may contain one or more heteroatoms, more preferably a C₁-C₃₀ hydrocarbon group that may contain one or more heteroatoms, and particularly preferably a C₁-C₈ hydrocarbon group that may contain one or more heteroatoms. Examples of the hydrocarbon group that may contain one or more heteroatoms include a methyl group, an ethyl group, a propyl group, an isopropyl group, a butyl group, a sec-butyl group, a tert-butyl group, a pentyl group, a hexyl group, an octyl group, a decyl group, a dodecyl group, an allyl group, a benzyl group, a cyclohexyl group, an adamantyl group, a phenyl group, a 2,6-diisopropylphenyl group, a 2,4,6-trimethylphenyl group, a 2-methoxyethyl group, a 2-ethoxyethyl group, a 2-(dimethylamino)ethyl group, and the like. Preferred are a methyl group, an ethyl group, a propyl group, an isopropyl group, a tert-butyl group, an octyl group, a cyclopentyl group, a cyclohexyl group, and a 2,4,6-trimethylphenyl group; more preferred are a methyl group, an ethyl group, an isopropyl group, a tert-butyl group, an octyl group, and a phenyl group; and particularly preferred are a methyl group, an isopropyl group, a tert-butyl group, an octyl group, and a phenyl group.

In the present invention, the nitrogen-containing compound (4) is preferably a nitrogen-containing compound represented by formula (4-1), (4-2), or (4-3), and particularly preferably a nitrogen-containing compound represented by formula (4-1).

Formula (4-1) : R⁹-Q (4-1)

wherein Q is as defined above, and R⁹ is a hydrocarbon group optionally substituted with one or more halogen atoms or a hydrocarbon group optionally substituted with one or more heteroatoms.

Formula (4-2): **Q-R¹⁰-Q (4-2)**

wherein each Q is as defined above, and R¹⁰ is a divalent hydrocarbon group optionally substituted with one or more halogen atoms or a divalent hydrocarbon group optionally substituted with one or more heteroatoms. 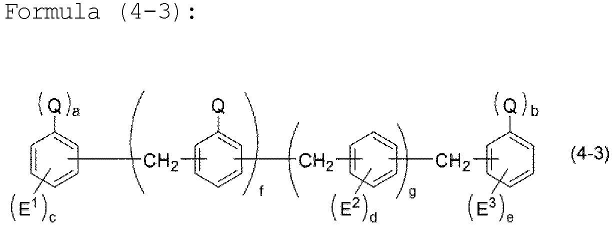 wherein each Q is the same or different and is as defined above; E¹, E², and E³ are each independently a hydrocarbon group optionally substituted with one or more halogen atoms, a hydrocarbon group optionally substituted with one or more heteroatoms, a halogen atom, a dialkylamino group, an alkoxy group, an aryloxy group, a nitro group, a cyano group, a sulfonyl group, an (alkylamino)carbonylamino group, a (dialkylamino)carbonylamino group, or an isocyanate group; f and g are each independently an integer of 0 to 4; a and b are each 0 or 1; and c, d, and e are each independently an integer of 0 to 4; provided that when f is 0, at least one of a or b is 1.

When Q is an -NCO group, the nitrogen-containing compound (4) is an isocyanate compound represented by the following formula (4a) (hereinafter referred to as "the isocyanate compound (4a)"). When Q is an -NHCO₂R⁸ group, the nitrogen-containing compound (4) is a urethane compound represented by the following formula (4b) (hereinafter referred to as "the urethane compound (4b)"). 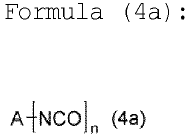 wherein A and n are as defined above. 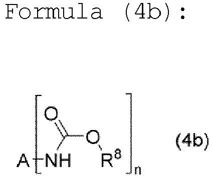 wherein A, n, and R⁸ are as defined above.

When Q is an -NCO group in formulas (4-1), (4-2), and (4-3), the nitrogen-containing compounds represented by formulas (4-1), (4-2), and (4-3) have structures represented by formulas (4a-1), (4a-2), and (4a-3), respectively.

Formula (4a-1): **R⁹-NCO (4a-1)**

wherein R⁹ is as defined above.

Formula (4a-2): **OCN-R¹⁰-NCO (4a-2)**

wherein R¹⁰ is as defined above. 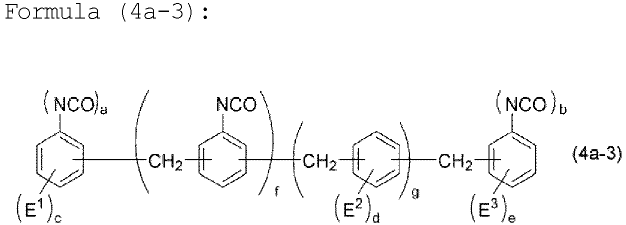 wherein E¹, E², E³, a, b, c, d, e, f, and g are as defined above.

In the present invention, a polymer such as polymethylene polyphenyl polyisocyanate (polymeric MDI) can also be used as the isocyanate compound (4a).

In formula (4a-1), R⁹ is a hydrocarbon group optionally substituted with one or more halogen atoms or a hydrocarbon group optionally substituted with one or more heteroatoms, preferably a C₁-C₅₀ hydrocarbon group optionally substituted with one or more halogen atoms or a C₁-C₅₀ hydrocarbon group optionally substituted with one or more heteroatoms, more preferably a C₁-C₃₀ hydrocarbon group optionally substituted with one or more halogen atoms or a C₁-C₃₀ hydrocarbon group optionally substituted with one or more heteroatoms, and particularly preferably a C₁-C₁₂ hydrocarbon group optionally substituted with one or more halogen atoms or a C₁-C₁₂ hydrocarbon group optionally substituted with one or more heteroatoms. The hydrocarbon group is preferably an aromatic hydrocarbon group, such as an aryl group or an arylalkyl group.

Specific examples of the hydrocarbon group optionally substituted with one or more halogen atoms or the hydrocarbon group optionally substituted with one or more heteroatoms represented by R⁹ include a methyl group, an ethyl group, a propyl group, an isopropyl group, a butyl group, a sec-butyl group, a tert-butyl group, a pentyl group, a hexyl group, a heptyl group, a decyl group, a dodecyl group, an octadecyl group, a cyclopropyl group, a cyclopentyl group, a cyclohexyl group, a phenyl group, a chlorophenyl group, a naphthyl group, a benzyl group, a phenethyl group, a tolyl group, an allyl group, and the like. Preferred are a benzyl group and a phenyl group.

Examples of halogen atoms in the hydrocarbon group optionally substituted with one or more halogen atoms or the hydrocarbon group optionally substituted with one or more heteroatoms represented by R⁹ include fluorine, chlorine, bromine, iodine, and the like. The hydrocarbon group of R⁹ may be substituted with one or more heteroatoms, such as oxygen, nitrogen, and sulfur. When the hydrocarbon group of R⁹ is substituted with a heteroatom, such as oxygen, nitrogen, or sulfur, the hydrocarbon group has a group, such as -O-, -N<, -S-, or -SO₂-, and the hydrocarbon chain is interrupted by such a group.

In formula (4a-2), R¹⁰ is a divalent hydrocarbon group optionally substituted with one or more halogen atoms or a divalent hydrocarbon group optionally substituted with one or more heteroatoms, preferably a C₁-C₁₀₀ divalent hydrocarbon group optionally substituted with one or more halogen atoms or a C₁-C₁₀₀ divalent hydrocarbon group optionally substituted with one or more heteroatoms, more preferably a C₁-C₅₀ divalent hydrocarbon group optionally substituted with one or more halogen atoms or a C₁-C₅₀ divalent hydrocarbon group optionally substituted with one or more heteroatoms, and particularly preferably a C₁-C₃₀ divalent hydrocarbon group optionally substituted with one or more halogen atoms or a C₁-C₃₀ divalent hydrocarbon group optionally substituted with one or more heteroatoms. The hydrocarbon group is preferably a divalent aromatic hydrocarbon group, such as an arylene group, an arylalkylene group, or an arylenealkylene group. Specific examples of the divalent hydrocarbon group optionally substituted with one or more halogen atoms or the divalent hydrocarbon group optionally substituted with one or more heteroatoms include alkylene groups, such as a methylene group, a dimethylmethylene group, an ethylene group, an n-propylene group, an n-butylene group, an n-pentylene group, an n-hexylene group, an n-heptylene group, an n-octylene group, an n-nonylene group, an n-decylene group, an n-dodecylene group, an n-octadecylene group, and a cyclohexylene group; arylene groups, such as a phenylene group, a 2-methylphenylene group, a 2,6-dimethylphenylene group, a 2,4-dimethylphenylene group, a 2,3-dimethylphenylene group, and a naphthylene group; arylalkylene groups, such as a phenylmethylene group, a phenylethylene group, a 1-phenylpropylene group, a 2-phenylpropylene group, a 1-phenylbutylene group, 2-phenylbutylene group, a naphthylmethylene group, and a naphthylethylene group; arylenealkylene groups obtained by suitably combining the above alkylene groups and arylene groups; and the like. These divalent hydrocarbon groups may be repeated or combined to constitute one divalent hydrocarbon group.

When the divalent hydrocarbon group represented by R¹⁰ is substituted with one or more halogen atoms, some or all of the hydrogen atoms bonded to the carbon atom(s) in the divalent hydrocarbon group may be replaced by halogen atom(s). Examples of halogen atoms include fluorine, chlorine, bromine, iodine, and the like. Specific examples of divalent hydrocarbon groups substituted with one or more halogen atoms include a 1-chloro-3,5-phenylene group, a 2-chloro-1,4-phenylene group, a 1-bromo-3,5-phenylene group, a 1,4-dichloro-3,5-phenylene group, a 1,2,4,5-tetrachloro-3,6-phenylene group, a 1-chloro-4,5-naphthylene group, and the like. The divalent hydrocarbon group of R¹⁰ may be substituted with one or more heteroatoms, such as oxygen, nitrogen, and sulfur. When the divalent hydrocarbon group of R¹⁰ is substituted with a heteroatom, such as oxygen, nitrogen, or sulfur, the hydrocarbon group has a group, such as -O-, -N<, -S-, or -SO₂-, and the hydrocarbon chain is interrupted by such a group.

In formula (4a-3), E¹, E², and E³ are each independently a hydrocarbon group optionally substituted with one or more halogen atoms, a hydrocarbon group optionally substituted with one or more heteroatoms, a halogen atom, a dialkylamino group, an alkoxy group, an aryloxy group, a nitro group, a cyano group, a sulfonyl group, an (alkylamino)carbonylamino group, a (dialkylamino)carbonylamino group, or an isocyanate group; preferably a hydrocarbon group optionally substituted with one or more halogen atoms or a hydrocarbon group optionally substituted with one or more heteroatoms, an (alkylamino)carbonylamino group, a (dialkylamino)carbonylamino group, or an isocyanate group; and more preferably an (alkylamino)carbonylamino group or a (dialkylamino)carbonylamino group.

The hydrocarbon group optionally substituted with one or more halogen atoms or the hydrocarbon group optionally substituted with one or more heteroatoms is preferably a C₁-C₅₀ hydrocarbon group optionally substituted with one or more halogen atoms or a C₁-C₅₀ hydrocarbon group optionally substituted with one or more heteroatoms, more preferably a C₁-C₃₀ hydrocarbon group optionally substituted with one or more halogen atoms or a C₁-C₃₀ hydrocarbon group optionally substituted with one or more heteroatoms, and particularly preferably a C₁-C₁₂ hydrocarbon group optionally substituted with one or more halogen atoms or a C₁-C₁₂ hydrocarbon group optionally substituted with one or more heteroatoms.

When E¹, E², and E³ are each independently a halogen atom, examples of halogen atoms include fluorine, chlorine, bromine, iodine, and the like.

Examples of halogen atoms in the hydrocarbon group optionally substituted with one or more halogen atoms represented by E¹, E², or E³ include fluorine, chlorine, bromine, iodine, and the like. The hydrocarbon group of E¹, E², or E³ may be substituted with one or more heteroatoms, such as oxygen, nitrogen, and sulfur. When the hydrocarbon group of E¹, E², or E³ is substituted with a heteroatom, such as oxygen, nitrogen, or sulfur, the hydrocarbon group has a group, such as -O-, -N<, -S-, or -SO₂-, and the hydrocarbon chain is interrupted by such a group.

Examples of the alkyl moiety of the above dialkylamino groups, alkoxy groups, (alkylamino)carbonylamino groups, and (dialkylamino)carbonylamino groups include linear or branched C₁-C₆ alkyl groups, such as methyl, ethyl, n-propyl, isopropyl, n-butyl, isobutyl, sec-butyl, tert-butyl, and n-pentyl. The number of carbon atoms in the alkyl group is preferably 1 to 3, and more preferably 1 or 2.

Examples of the aryl moiety of the above aryloxy groups include C₆-C₁₀ aryl groups. Specific examples include a phenyl group, a naphthyl group, and the like.
f and g are each independently an integer of 0 to 4. a and b are each 0 or 1, and c, d, and e are each independently an integer of 0 to 4. However, when f is 0, at least one of a or b is 1.

Specific examples of the isocyanate compound (4a) are shown below. However, the present invention is not limited thereto. 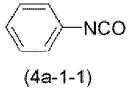 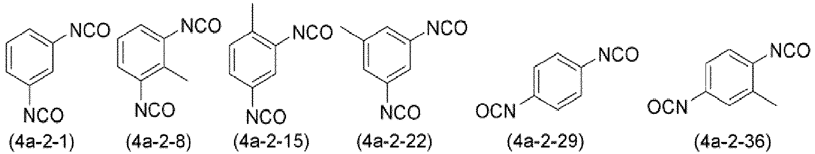 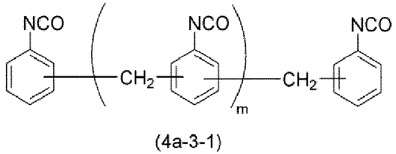 wherein m is as defined above.

The isocyanate compound (4a) is preferably a compound represented by formula (4a-1-1), (4a-2-8), (4a-2-15), or (4a-3-1).

The isocyanate compounds (4a) may be used singly or as a mixture of two or more.

When Q is -NHCO₂R⁸ group in formulas (4-1), (4-2), and (4-3), the nitrogen-containing compounds represented by formulas (4-1), (4-2), and (4-3) have structures represented by formulas (4b-1), (4b-2), and (4b-3), respectively. 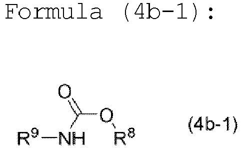 wherein R⁸ and R⁹ are as defined above. 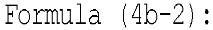 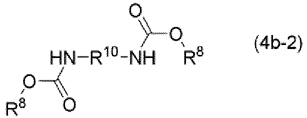 wherein R⁸ and R¹⁰ are as defined above. 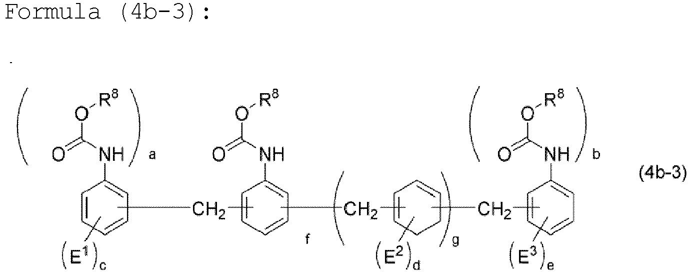 wherein R⁸, E¹, E², E³, a, b, c, d, e, f, and g are as defined above.

Specific examples of the urethane compound (4b) are shown below. However, the present invention is not limited thereto. In the following specific examples, Et represents an ethyl group, Bu represents an n-butyl group, t-Bu represents a t-butyl group, Oct represents an n-octyl group, and Ph represents a phenyl group.

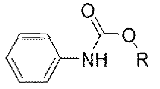

| R | |
|---|---|
| CH₃ | (4b-1-1) |
| Bu | (4b-1-2) |
| t-Bu | (4b-1-3) |
| Oct | (4b-1-4) |
| CH₂CH(Et)Bu | (4b-1-5) |
| C₁₂H₂₅ | (4b-1-6) |
| CH₂Ph | (4b-1-7) |

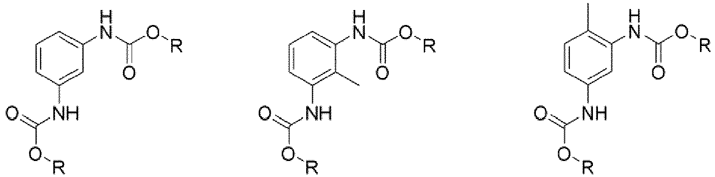

| R | |
|---|---|
| CH₃ | (4b-2-1) |
| Bu | (4b-2-2) |
| t-Bu | (4b-2-3) |
| Oct | (4b-2-4) |
| CH₂CH(Et)Bu | (4b-2-5) |
| C₁₂H₂₅ | (4b-2-6) |
| CH₂Ph | (4b-2-7) |

| R | |
|---|---|
| CH₃ | (4b-2-8) |
| Bu | (4b-2-9) |
| t-Bu | (4b-2-10) |
| Oct | (4b-2-11) |
| CH₂CH(Et)Bu | (4b-2-12) |
| C₁₂H₂₅ | (4b-2-13) |
| CH₂Ph | (4b-2-14) |

| R | |
|---|---|
| CH₃ | (4b-2-15) |
| Bu | (4b-2-16) |
| t-Bu | (4b-2-17) |
| Oct | (4b-2-18) |
| CH₂CH(Et)Bu | (4b-2-19) |
| C₁₂H₂₅ | (4b-2-20) |
| CH₂Ph | (4b-2-21) |

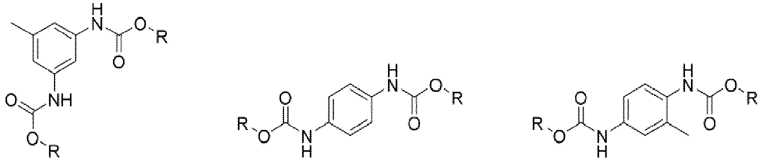

| R | |
|---|---|
| CH₃ | (4b-2-22) |
| Bu | (4b-2-23) |
| t-Bu | (4b-2-24) |
| Oct | (4b-2-25) |
| CH₂CH(Et)Bu | (4b-2-26) |
| C₁₂H₂₅ | (4b-2-27) |
| CH₂Ph | (4b-2-28) |

| R | |
|---|---|
| CH₃ | (4b-2-29) |
| Bu | (4b-2-30) |
| t-Bu | (4b-2-31) |
| Oct | (4b-2-32) |
| CH₂CH(Et)Bu | (4b-2-33) |
| C₁₂H₂₅ | (4b-2-34) |
| CH₂Ph | (4b-2-35) |

| R | |
|---|---|
| CH₃ | (4b-2-36) |
| CH₃ | (4b-2-37) |
| Bu | (4b-2-38) |
| Oct | (4b-2-39) |
| CH₂CH(Et)Bu | (4b-2-40) |
| C₁₂H₂₅ | (4b-2-41) |
| CH₂Ph | (4b-2-42) |

| 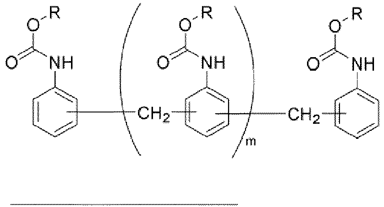 | |
|---|---|
| R | |
| CH₃ | (4b-3-1) |
| CH₃ | (4b-3-2) |
| Bu | (4b-3-3) |
| Oct | (4b-3-4) |
| CH₂CH(Et)Bu | (4b-3-5) |
| C₁₂H₂₅ | (4b-3-6) |
| CH₂Ph | (4b-3-7) |

Preferable examples of the urethane compound (4b) include compounds represented by formulas (4b-1-1) to (4b-1-7), (4b-2-8) to (4b-2-21), and (4b-3-1) to (4b-3-7). Particularly preferable examples of the urethane compound (4b) include compounds represented by formulas (4b-1-1), (4b-2-8), and (4b-3-1).

The urethane compound (4b) used as a starting material may be a commercial product, or may be produced by a known method.

The amidate compound represented by formula (5) (hereinafter referred to as "the amidate compound (5)") is described below.

In formula (5), A, R¹, R², R³, R⁴, and n are as defined above.

The amidate compound (5) is preferably an amidate compound represented by formula (5-1), (5-2), or (5-3). 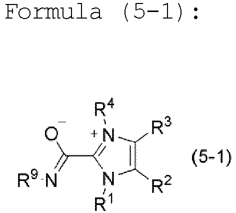 wherein R¹, R², R³, R⁴, and R⁹ are as defined above. 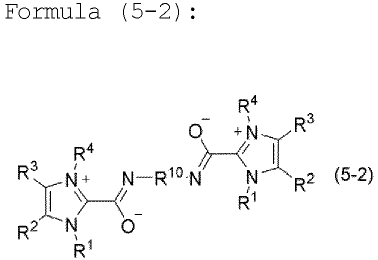 wherein R¹, R², R³, R⁴, and R¹⁰ are as defined above. 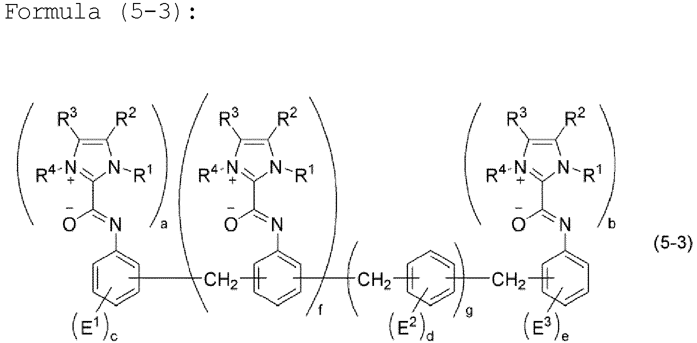 wherein R¹, R², R³, R⁴, E¹, E², E³, a, b, c, d, e, f, and g are as defined above.

In formula (5-1), R¹, R², R³, R⁴, and R⁹ are as defined above.

In formula (5-2), R¹, R², R³, R⁴, and R¹⁰ are as defined above.

In formula (5-3), R¹, R², R³, R⁴, E¹, E², E³, a, b, c, d, e, f, and g are as defined above.

Next, specific examples of the amidate compound (5) are shown below. However, the present invention is not limited thereto. In the following specific examples, Et represents an ethyl group, Pr represents an n-propyl group, Bu represents an n-butyl group, Oct represents an n-octyl group, and t-Oct represents a 1,1,3,3-tetramethylbutyl group.

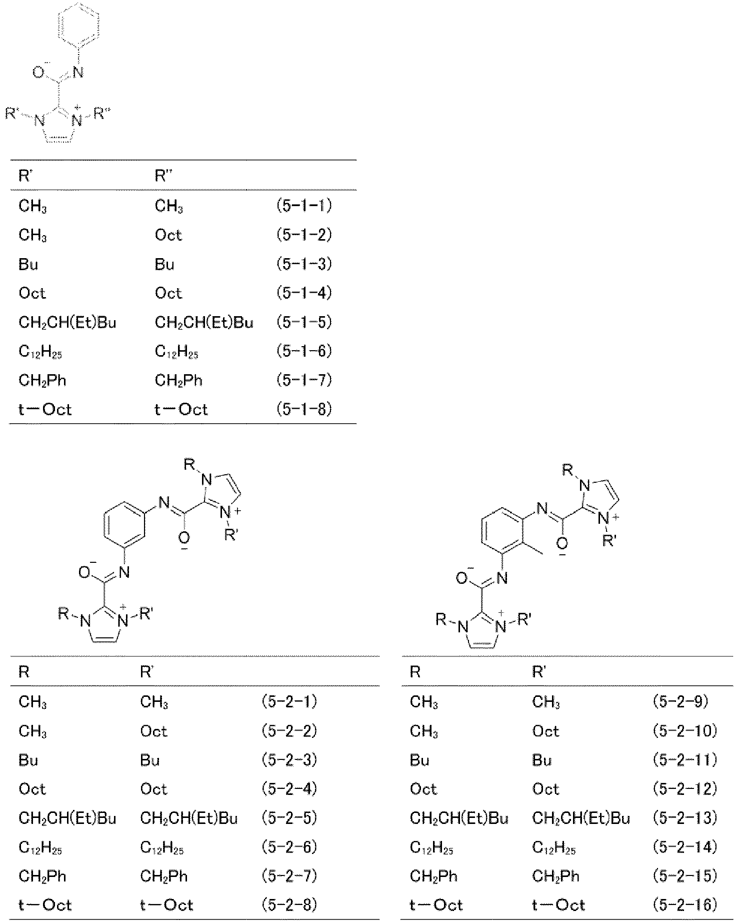

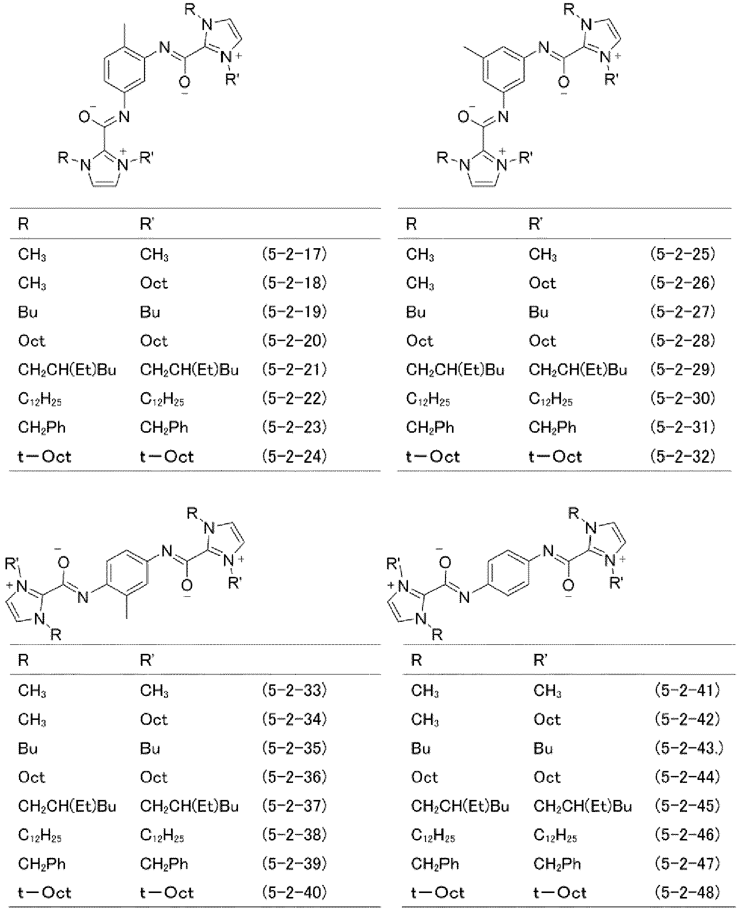

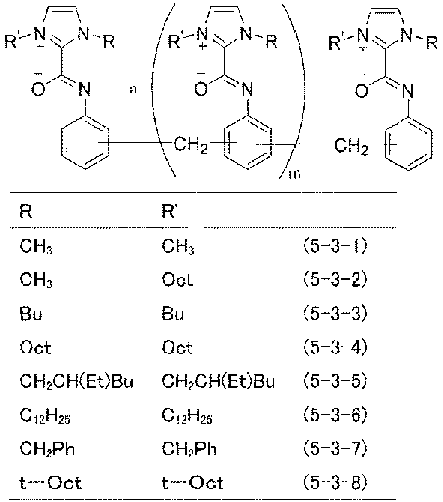

Preferable examples of the amidate compound (5) include compounds represented by formulas (5-1-1) to (5-1-8), (5-2-9) to (5-2-24), and (5-3-1) to (5-3-8). Particularly preferable examples of the amidate compound (5) include compounds represented by formulas (5-1-4), (5-1-5), (5-1-8), (5-2-13), and (5-3-5).

When the amidate compound (5) is an isomer, such as an enantiomer, a stereoisomer, or a regioisomer, the amidate compound (5) includes a mixture of any isomers, unless the isomer is specified. For example, when the amidate compound (5) is an enantiomer, the amidate compound (5) also includes enantiomers divided from the racemic form. These isomers can be obtained as single compounds by conventionally known separation methods (concentration, solvent extraction, column chromatography, recrystallization, etc.).

In step 2, the amount of the nitrogen-containing compound (4) used is generally such that the group represented by Q in the nitrogen-containing compound (4) is allowed to react in an amount of 0.8 mol or more, preferably 1 to 3 mol, per mol of the total of the carboxylate compound (3a) and the compounds (3b) to (3f) contained in the reaction product (A). The total of the carboxylate compound (3a) and the compounds (3b) to (3f) contained in the reaction product (A) may be calculated from the amount of the imidazolium carboxylic acid salt (1) used in step 1.

When the reaction product (A) contains the carboxylate compound (3a), the group represented by Q in the nitrogen-containing compound (4) is allowed to react in an amount of 0.8 mol or more, preferably 1 to 3 mol, per mole of the carboxylate compound (3a).

In this case, when the reaction product (A) contains the compounds (3b) to (3f), the amount of the nitrogen-containing compound (4) used is determined assuming that the reaction product compounds (3b) to (3f) are also regarded as the carboxylate compound (3a).

The reaction temperature in step 2 is not particularly limited, and is generally -10°C or higher, preferably 0 to 200°C, and more preferably 20 to 150°C; and the reaction time is generally 0.1 to 24 hours, and preferably 1 to 10 hours.

When a solvent is used, examples of solvents include aromatic hydrocarbon solvents, such as toluene, benzene, and xylene; aliphatic hydrocarbon solvents, such as methylcyclohexane, cyclohexane, hexane, heptane, and octane; halogenated hydrocarbon solvents, such as butyl chloride and 1,2-dichloroethane; halogenated aromatic hydrocarbon solvents, such as chlorobenzene; ester solvents, such as ethyl acetate and butyl acetate; ketone solvents, such as methyl ethyl ketone and 4-methyl-2-pentanone; and the like. Preferred are aromatic hydrocarbon solvents, halogenated aromatic hydrocarbon solvents, ester solvents, and ketone solvents; and particularly preferred are toluene, xylene, chlorobenzene, butyl acetate, and 4-methyl-2-pentanone. The solvents can be used as a mixture of two or more, if necessary. When the reaction product (A) contains the solvent used in step 1, the solvent can be used as a solvent in step 2, and further a solvent mentioned above may be added. In this case, a solvent different from that in step 1 may be used.

When a solvent is used, the amount of solvent used is generally 50 parts by mass or less, and preferably 0.1 parts by mass or more and 35 parts by mass or less, per part by mass of the total mass of the carboxylate compound (3a) and the compounds (3b) to (3f). In another embodiment, the amount of solvent used is generally 50 parts by mass or less, and preferably 0.1 parts by mass or more and 35 parts by mass or less, per part by mass of the components other than the solvent in the reaction product (A) in step 1.

The components other than the solvent in the reaction product (A) indicate the concentrated residue obtained by removing the solvent from the reaction liquid obtained in step 1. The weight of the concentrated residue may be determined by actually removing the solvent from the reaction liquid by concentration or filtration or by calculating the weight of the solvent in the reaction liquid by ¹H-NMR or GC analysis, and then subtracting the calculated solvent weight from the weight of the reaction liquid.

The reaction may be performed, if necessary, in an inert gas atmosphere, such as nitrogen, argon, or helium, which do not affect the reaction.

After completion of the reaction, the amidate compound (5) can be obtained by removing the solvent by concentrating or filtering the reaction liquid, and may be purified by recrystallization, column separation, etc., if necessary.

### Examples

The present invention is described in detail below based on Examples; however, the present invention is not limited thereto. In the Examples, Bruker AV400 was used for ¹H-NMR measurement, which was performed at 400 MHz. In the Examples, wt% indicates mass percent concentration.

### Production Example 1: Synthesis of [DOI][OAc]

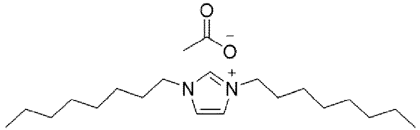

1049.9 g (8.12 mol) of octylamine was placed in a 3-L four-necked reactor purged with nitrogen and heated to 80°C. Subsequently, a mixture of 366.0 g (6.09 mol) of acetic acid and 290.4 g of 42 wt% formalin aqueous solution (formaldehyde pure content: 4.06 mol) was added dropwise over a period of 1 hour. After dropwise addition, the mixture in the reactor was stirred for 30 minutes and cooled to 40°C. 581.0 g of 41 wt% glyoxal aqueous solution (glyoxal pure content: 4.06 mol) was added dropwise to the cooled mixture over a period of 30 minutes, and the resulting mixture was stirred for 5 hours. After stirring, the resulting reaction solution was concentrated under reduced pressure to give 1582.0 g of [DOI][OAc] represented by the above formula. The results of ¹H-NMR analysis of the resulting [DOI][OAc] using tetralin added thereto as an internal standard revealed that 1224.9 g (3.47 mol; yield: 85.5%) of [DOI][OAc] was contained. The ¹H-NMR analysis results of [DOI][OAc] are shown below. ¹H-NMR(DMSO-d₆)δ(ppm)=9.32 (s, 1H), 7.80 (s, 2H), 4.17 (t, J=9.6 Hz, 4H), 1.78 (m, 4H), 1.63 (s, 3H), 1.23 (m, 20H), 0.85 (t, J=6.4 Hz, 6H)

### Production Example 2: Synthesis of [DBI][OAc]

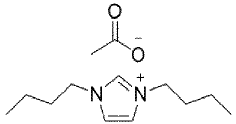

40 g (0.55 mol) of butylamine was placed in a 200-mL four-necked reactor purged with nitrogen and cooled to 0°C. Subsequently, a mixture of 24.6 g (0.41 mol) of acetic acid and 20.8 g (0.28 mol) of 40 wt% formalin aqueous solution was added dropwise over a period of 2 hours. After dropwise addition, the mixture in the reactor was stirred for 30 minutes. 39.6 g (0.27 mol) of 40 wt% glyoxal aqueous solution was added dropwise to the mixture over a period of 30 minutes, followed by stirring for 16 hours. After stirring, 100 mL of heptane was added to the resulting reaction solution to perform liquid separation, and the operation of removing the heptane layer was performed twice to obtain a brown solution. The resulting brown solution was concentrated under reduced pressure to give 75.8 g (0.27 mol; yield: 98%) of [DBI][OAc] represented by the above formula. The ¹H-NMR analysis results of [DBI][OAc] are shown below. ¹H-NMR(DMSO-d₆)δ(ppm)=9.53 (s, 1H), 7.82 (s, 2H), 4.19 (t, J=6.8 Hz, 4H), 1.81 (m, 4H), 1.68 (s, 4.5H), 1.29 (m, 4H), 0.91 (t, J=7.2 Hz, 6H)

### Production Example 3: Synthesis of [D2EHI][OAc]

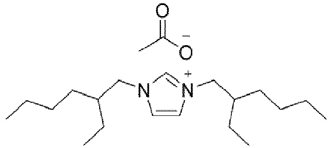

750.0 g (5.80 mol) of 2-ethylhexylamine was placed in a 2-L four-necked reactor purged with nitrogen, and a mixture of 261.3 g (4.35 mol) of acetic acid and 217.8 g (2.90 mol) of 40 wt% formalin aqueous solution was added dropwise over a period of 1 hour and 30 minutes. After dropwise addition, the mixture in the reactor was stirred for 30 minutes. 421.0 g (2.90 mol) of 40 wt% glyoxal aqueous solution was added dropwise to the mixture over a period of 20 minutes, followed by stirring for 5 hours. The resulting reaction solution was concentrated under reduced pressure to give 1219.1 g (2.89 mol; yield: 99%) of [D2EHI][OAc] represented by the above formula. The ¹H-NMR analysis results of [D2EHI][OAc] are shown below.
¹H-NMR(DMSO-d₆)δ(ppm)=9.35 (s, 1H), 7.82 (s, 2H), 4.15 (d, J= 7.2 Hz, 4H), 1.84 (m, 2H), 1.71 (s, 3H), 1.25 (m, 16H), 0.87 (t, J=7.2 Hz, 12H)

### Production Example 4: Synthesis of [DtOctI][OAc]

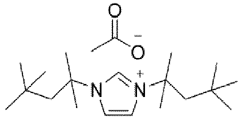

6.97 g (0.116 mol) of acetic acid, 5.80 g of 40 wt% formalin aqueous solution (formaldehyde pure content: 0.077 mol), and 11.2 g of 40 wt% glyoxal aqueous solution (glyoxal pure content: 0.077 mol) were placed in a 100-ml three-necked reactor purged with nitrogen, and the mixture was heated to 50°C. Subsequently, 20.00 g (0.154 mol) of 1,1,3,3-tetramethylbutylamine was added dropwise to the mixture in the reactor over a period of 2 hours, and the resulting mixture was stirred for 2 hours. After stirring, the resulting reaction solution was concentrated under reduced pressure to give 24.83 g (0.069 mol; yield: 90%) of [DtOctI][OAc] represented by the above formula. The ¹H-NMR analysis results of [DtOctI][OAc] are shown below.
¹H-NMR(CDCl₃)δ(ppm)=10.63 (s, 1H), 7.34 (s, 2H), 2.04 (s, 3H), 2.04 (s, 4H), 1.83 (s, 12H), 0.87 (s, 18H)

### Example 1: Synthesis of DOIm_PI

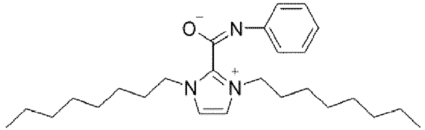

### (Step 1) Production of 1,3-di-n-octylimidazolium-2-carboxylate

1070.1 g of the [DOI][OAc] ([DOI][OAc] pure content: 828.6 g (2.35 mol)) obtained in Production Example 1, 1069.5 g of toluene, and 756.0 g (8.39 mol) of dimethyl carbonate were placed in a 5-L pressure-resistant container, followed by purging with nitrogen. Subsequently, the mixture was stirred at 120°C for 15 hours. After stirring, the resulting reaction mixture was cooled. The cooled reaction mixture was concentrated under reduced pressure to 1043.5 g to remove excess dimethyl carbonate and methyl acetate as a by-product, and toluene was added thereto to make 2058.6 g. The results of ¹H-NMR analysis of the resulting toluene solution using dimethyl sulfoxide as an internal standard revealed that 339.3 g (1.01 mol; yield: 42.8%) of 1,3-di-n-octylimidazolium-2-carboxylate was obtained. It was also found that the resulting toluene solution contained 128.0 g (0.38 mol; yield: 16.2%) of 1,3-di-n-octylimidazolium-4-carboxylate and 211.7 g (0.57 mol; yield: 24.4%) of 1,3-di-n-octylimidazolium methylcarbonate salt.

The ¹H-NMR analysis results of 1,3-di-n-octylimidazolium-2-carboxylate are shown below. ¹H-NMR(CDCl₃)δ(ppm)=7.21 (s, 2H), 4.55 (t, J=7.6 Hz, 4H), 1.78 (m, 4H), 1.31 (m, 20H), 0.88 (t, J=6.4 Hz, 6H)

The ¹H-NMR analysis results of 1,3-di-n-octylimidazolium-4-carboxylate are shown below. ¹H-NMR(CDCl₃)δ(ppm)=7.49 (s, 1H), 4.73 (t, J=7.2 Hz, 2H), 4.26 (t, J=7.2 Hz, 2H), 1.78 (m, 4H), 1.31 (m, 20H), 0.88 (t, J=6.4 Hz, 6H)

The ¹H-NMR analysis results of 1,3-di-n-octylimidazolium methylcarbonate salt are shown below. ¹H-NMR(CDCl₃)δ(ppm)=7.44 (s, 2H), 4.32 (t, J=7.2 Hz, 4H), 3.39 (s, 3H), 1.78 (m, 4H), 1.31 (m, 20H), 0.88 (t, J=6.4 Hz, 6H)

### (Step 2)

1700.2 g of the toluene solution obtained in step 1 and 807.4 g of toluene were placed in a 3-L four-necked reactor purged with nitrogen and heated under reflux. While heating under reflux, 229.7 g (1.93 mol) of phenyl isocyanate was added dropwise over a period of 2 hours, and the mixture was stirred for 10 hours. After stirring, the resulting reaction mixture was concentrated to 1433.1 g, and 761.0 g of heptane was added. The resulting mixture was heated to 50°C to dissolve all of the solids, thereby obtaining a heptane solution. The heptane solution was cooled from 50°C to 30°C to precipitate crystals, followed by stirring at 30°C for 1 hour. Subsequently, the heptane solution was cooled to 10°C over a period of 2 hours at 10°C per hour and stirred at 10°C for another 1 hour to obtain a slurry liquid. The resulting slurry liquid was filtered to give 426.8 g (1.04 mol; overall yield from step 1: 62.2%) of a compound (DOIm_PI) represented by the above formula as a pale yellow solid. The ¹H-NMR analysis results of DOIm_PI are shown below.
¹H-NMR(DMSO-d₆)δ(ppm)=9.32 (s, 1H), 7.80 (s, 2H), 4.17 (t, J=9.6 Hz, 4H), 1.78 (m,4H), 1.63 (s, 3H), 1.23 (m, 20H), 0.85 (t, J=6.4 Hz, 6H)

### Example 2: Synthesis of DBIm_PI

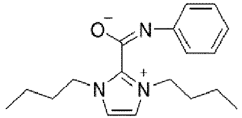

### (Step 1)

30.0 g (pure content: 0.11 mol) of the obtained in Production Example 2, 44.8 g (0.50 mol) of dimethyl carbonate, and 30.0 g of toluene were placed in a 180-mL pressure-resistant container, followed by purging with nitrogen. Subsequently, the mixture was stirred at 120°C for 7 hours. After stirring, the resulting reaction mixture was concentrated under reduced pressure to obtain 25.2 g of a brown liquid. The results of ¹H-NMR analysis of the resulting brown liquid and calculation from the integral ratio revealed that 9.8 g (0.04 mol; yield: 39.2%) of 1,3-di-n-butylimidazolium-2-carboxylate was obtained. It was also found that the resulting toluene solution contained 1.8 g (0.008 mol; yield: 7.4%) of 1,3-di-n-butylimidazolium-4-carboxylate and 13.8 g (0.05 mol; yield: 48.5%) of 1,3-di-n-butylimidazolium methylcarbonate salt. 25.2 g of chlorobenzene was added to the resulting brown liquid to prepare a chlorobenzene solution.

The ¹H-NMR analysis results of 1,3-di-n-butylimidazolium-2-carboxylate are shown below. ¹H-NMR(DMSO-d₆)δ(ppm)=7.71 (s, 2H), 4.45 (t, J=7.2 Hz, 4H), 1.81 (m, 4H), 1.28 (m, 4H), 0.89 (m, 6H)

The ¹H-NMR analysis results of 1,3-di-n-butylimidazolium-4-carboxylate are shown below. ¹H-NMR(DMSO-d₆)δ(ppm)=9.45 (s, 1H), 7.60 (s, 1H), 4.59 (t, J=7.2 Hz, 2H), 4.15 (t, J=7.2 Hz, 2H), 1.81 (m, 4H), 1.28 (m, 4H), 0.89 (m, 6H)

The ¹H-NMR analysis results of 1,3-di-n-butylimidazolium methylcarbonate salt are shown below. ¹H-NMR(DMSO-d₆)δ(ppm)=9.73 (s, 1H), 7.89 (s, 2H), 4.22 (t, J=7.2 Hz, 4H), 3.17 (s, 3H), 1.81 (m, 4H), 1.28 (m, 4H), 0.89 (m, 6H)

### (Step 2)

40.0 g of the chlorobenzene solution obtained in step 1, 40.0 g of chlorobenzene, and 13.4 g (0.10 mol) of methyl N-phenylcarbamate were placed in a 180-mL three-necked reactor purged with nitrogen and heated under reflux for 5 hours. After heating under reflux, the resulting reaction mixture was concentrated under reduced pressure to obtain 26.8 g of a dried residue. Subsequently, 17.1 g of butyl acetate was added to the dried residue, and the resulting mixture was heated to 60°C to dissolve all of the solids and then cooled to room temperature to give 5.8 g (19.4 mmol; overall yield from step 1: 21.8%) of a compound (DBIm_PI) represented by the above formula. The ¹H-NMR analysis results of DBIm_PI are shown below.
¹H-NMR(DMSO-d₆)δ(ppm)=7.60 (s, 2H), 7.42 (d, J=8.4 Hz, 2H), 7.14 (d, J=7.6 Hz, 2H), 6.80 (t, J=7.6 Hz, 1H), 4.48 (t, J=7.6 Hz, 4H), 1.80 (m, 4H), 1.30 (m, 4H), 0.91 (t, J=7.6 Hz, 6H)

### Example 3: Synthesis of D2HIm_PI

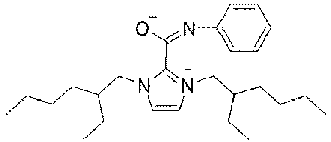

### (Step 1)

1104.2 g (pure content: 3.07 mol) of the [D2EHI][OAc] obtained in Production Example 3, 744.9 g (8.27 mol) of dimethyl carbonate, and 1104.2 g of toluene were placed in a 5-L pressure-resistant container, followed by purging with nitrogen. Subsequently, the mixture was heated to 120°C and then stirred for 12 hours. After stirring, the resulting reaction mixture was concentrated under reduced pressure to obtain 1095.8 g of a brown liquid. The results of ¹H-NMR analysis of the resulting brown liquid and calculation from the integral ratio revealed that 526.8 g (1.49 mol; yield: 46.6%) of 1,3-di-2-ethylhexylimidazolium-2-carboxylate was obtained. It was also found that the resulting toluene solution contained 142.4 g (0.423 mol; yield: 13.8%) of 1,3-di-2-ethylhexylimidazolium-4-carboxylate and 466.6 g (1.27 mol; yield: 41.2%) of 1,3-di-2-ethylhexylimidazolium methylcarbonate salt.

The ¹H-NMR analysis results of 1,3-di-2-ethylhexylimidazolium-2-carboxylate are shown below. ¹H-NMR(DMSO-d₆)δ(ppm)=7.70 (s, 2H), 4.38 (t, J=6.0 Hz, 4H), 1.84 (m, 2H), 1.25 (m, 16H), 0.87 (m, 12H)

The ¹H-NMR analysis results of 1,3-di-2-ethylhexylimidazolium-4-carboxylate are shown below. ¹H-NMR(DMSO-d₆)δ(ppm)=9.18 (s, 1H), 7.64 (s, 1H), 4.53 (t, J=6.0 Hz, 2H), 4.05 (t, J=6.0 Hz, 2H), 1.84 (m, 2H), 1.25 (m, 16H), 0.87 (m, 12H)

The ¹H-NMR analysis results of 1,3-di-2-ethylhexylimidazolium methylcarbonate salt are shown below. ¹H-NMR(DMSO-d₆)δ(ppm)=9.51 (s, 1H), 7.89 (s, 2H), 4.24 (t, J=7.6 Hz, 4H), 3.18 (s, 3H), 1.84 (m, 2H), 1.25 (m, 16H), 0.87 (m, 12H)

### (Step 2)

882.7 g of the brown liquid (total of 1,3-di-2-ethylhexylimidazolium-2-carboxylate and its equivalents, i.e., 1,3-di-2-ethylhexylimidazolium-4-carboxylate and 1,3-di-2-ethylhexylimidazolium methylcarbonate salt: 2.56 mol) obtained in step 1 and 2487.8 g of toluene were placed in a 5-L four-necked reactor purged with nitrogen and heated under reflux. Subsequently, 292.3 g (2.45 mol) of phenyl isocyanate was added dropwise to the liquid in the reactor over a period of 2 hours. After dropwise addition, a reaction was carried out under reflux for 13 hours and 30 minutes. After the reaction, the resulting reaction mixture was concentrated under reduced pressure to give 1078.6 g of a compound (D2HIm_PI) represented by the above formula. The results of ¹H-NMR analysis of the resulting D2HIm_PI using tetralin added thereto as an internal standard revealed that 990.9 g (2.41 mol; yield: 93.8%) of D2HIm_PI was contained. The results of ¹H-NMR analysis of D2HIm PI are shown below. ¹H-NMR(DMSO-d₆)δ(ppm)=7.59 (s, 2H), 7.39 (d, J=8.4 Hz, 2H), 7.12 (t, J=8.4 Hz, 2H), 6.76 (t, J=8.4 Hz ,1H), 4.44 (t, J=6.8 Hz, 4H), 1.86(m,2H), 1.25 (m, 16H), 0.82 (m, 12H)

### Example 4: Synthesis of D2HIm_crMDI

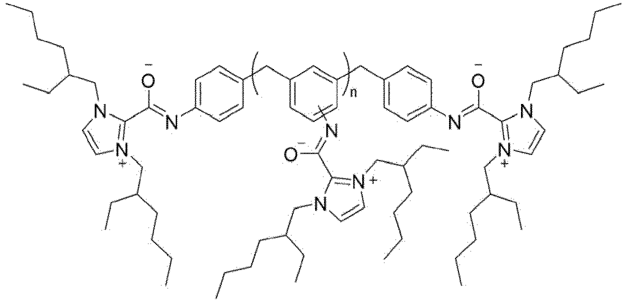

### (Step 1)

40.0 g (pure content: 99.5 mmol) of [D2EHI][OAc] obtained by the same production method as in Production Example 3 and 26.9 g (299 mmol) of dimethyl carbonate were placed in a 180-mL pressure-resistant container, followed by purging with nitrogen. Subsequently, the mixture in the pressure-resistant container was stirred at 120°C for 6 hours. After stirring, the resulting reaction mixture was concentrated under reduced pressure to obtain 35.3 g of a brown liquid. The results of ¹H-NMR internal standard analysis of the resulting brown liquid using dimethyl sulfone added thereto as an internal standard revealed that 7.5 g (0.02 mol; yield: 22.3%) of 1,3-di-2-ethylhexylimidazolium-2-carboxylate was obtained. It was also found that the resulting brown liquid contained 5.7 g (0.02 mol; yield: 17.1%) of 1,3-di-2-ethylhexylimidazolium-4-carboxylate and 18.3 g (0.05 mol; yield: 50.0%) of 1,3-di-2-ethylhexylimidazolium methylcarbonate salt.

### (Step 2)

30.2 g of toluene was placed in a 200-mL four-necked reactor purged with nitrogen and refluxed. While refluxing toluene, a mixture of 30.1 g of the brown liquid (total of 1,3-di-2-ethylhexylimidazolium-2-carboxylate and its equivalents, i.e., 1,3-di-2-ethylhexylimidazolium-4-carboxylate and 1,3-di-2-ethylhexylimidazolium methylcarbonate salt: 75.7 mmol) obtained in step 1 and 30.4 g of toluene was added dropwise to the reactor over a period of 2 hours. At the same time, a mixture of 10.6 g (NCO content: 83.1 mmol) of polymethylene polyphenyl polyisocyanate (Sumidur 44V20L produced by Sumika Covestro Urethane Co., Ltd., NCO content: 7.86 mmol/g) and 30.5 g of toluene was added dropwise to the reactor over a period of 2 hours, and the resulting mixture was stirred at reflux for 1 hour. After stirring, the resulting reaction mixture was concentrated under reduced pressure to give 37.4 g of a reaction product (D2HIm_crMDI) containing a compound represented by the above formula. The ¹H-NMR analysis results of the resulting reaction product are shown below.
¹H-NMR(CDCl₃)δ(ppm)=7.51-6.91 (m), 4.52-4.37 (m), 4.01-3.66 (m), 1.88 (br), 1.34-1.26 (m), 0.89-0.82 (m)

### Example 5: Synthesis of D2HIm_TDI

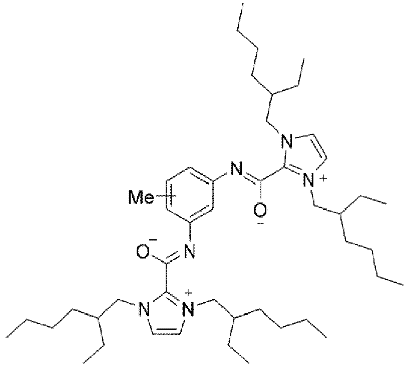

70.1 g (pure content: 0.17 mol) of [D2EHI][OAc] obtained by the same production method as in Production Example 3 and 53.9 g (0.60 mol) of dimethyl carbonate were placed in a 180-mL pressure-resistant container, followed by purging with nitrogen. Subsequently, the mixture in the pressure-resistant container was stirred at 120°C for 6 hours. After stirring, the resulting reaction mixture was concentrated under reduced pressure to obtain 67.7 g of a brown liquid. The results of ¹H-NMR internal standard analysis of the resulting brown liquid with dimethyl sulfone added thereto as an internal standard revealed that 5.3 g (0.02 mol; yield: 9.3%) of 1,3-di-2-ethylhexylimidazolium-2-carboxylate was obtained. It was also found that the resulting brown liquid contained 10.3 g (0.03 mol; yield: 18.1%) of 1,3-di-2-ethylhexylimidazolium-4-carboxylate and 41.8 g (0.11 mol; yield: 66.8%) of 1,3-di-2-ethylhexylimidazolium methylcarbonate salt, both of which are equivalents of 1,3-di-2-ethylhexylimidazolium-2-carboxylate.

### (Step 2)

30.3 g of toluene was placed in a 200-mL four-necked reactor purged with nitrogen and refluxed. Subsequently, while refluxing toluene, a mixture of 30.0 g of the brown liquid (total of 1,3-di-2-ethylhexylimidazolium-2-carboxylate and its equivalents, i.e., 1,3-di-2-ethylhexylimidazolium-4-carboxylate and 1,3-di-2-ethylhexylimidazolium methylcarbonate salt: 70.0 mmol) obtained in step 1 and 30.2 g of toluene was added dropwise to the reactor over a period of 2 hours. At the same time, a mixture of 6.7 g (38.4 mmol) of tolylene diisocyanate (produced by Tokyo Chemical Industry Co., Ltd., 2,4-tolylene diisocyanate: about 80%, 2,6-tolylene diisocyanate: about 20%) and 30.5 g of toluene was added dropwise to the reactor over a period of 2 hours, and the resulting mixture was stirred at reflux for 1 hour. After stirring, the resulting reaction mixture was concentrated under reduced pressure to give 31.2 g of a reaction product (D2HIm_TDI) containing a compound represented by the above formula. The ¹H-NMR analysis results of the resulting reaction product are shown below.
¹H-NMR(CDCl₃)δ(ppm)=7.28-6.88 (m, 5H), 4.59-4.43 (m, 8H), 2.24-2.17 (m, 3H), 1.92 (m, 4H), 1.37-1.27 (m, 32H), 0.9-0.83 (m, 24H)

### Example 6: Synthesis of DtOctIm_PI

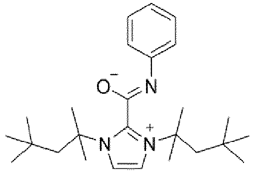

### (Step 1)

24.8 g (0.069 mol) of the [DtOctI][OAc] obtained in Production Example 4, 27.0 g (0.297 mol) of dimethyl carbonate, and 30.0 g of toluene were placed in a 180-mL pressure-resistant container, followed by purging with nitrogen. Subsequently, the mixture in the pressure-resistant container was stirred at 120°C for 6 hours. After stirring, the resulting reaction mixture was concentrated under reduced pressure to give a reaction product (A).

### (Step 2)

Half of the reaction product (A) obtained in step 1 and 40.0 g of chlorobenzene were placed in a 100-mL three-necked reactor purged with nitrogen and heated under reflux. After heating under reflux, the mixture was cooled to 60°C. A mixture of 3.9 g (0.032 mol) of phenyl isocyanate and 12.2 g of chlorobenzene was added dropwise thereto over a period of 10 minutes, and then the resulting mixture was stirred at 60°C for 1 hour. After stirring, the resulting reaction mixture was cooled to room temperature, and 50.0 g of heptane was added thereto, followed by filtration. The resulting residue was washed with 50.0 g of heptane to give 7.55 g (0.014 mmol; overall yield from step 1: 40.0%) of a compound (DtOctIm_PI) represented by the above formula. The ¹H-NMR analysis results of DtOctIm_PI are shown below.
¹H-NMR(CDCl₃)δ(ppm)=7.59 (d, J=9.2 Hz, 2H), 7.30 (t, J=7.6 Hz, 2H), 7.08 (s, 2H), 6.93 (t, J=7.2 Hz, 1H), 2.40 (m, 4H), 1.87 (m, 12H), 0.99 (s, 18H)

## Claims

1. A method for producing a carboxylate compound represented by the following formula (3a), the method comprising reacting an imidazolium carboxylic acid salt represented by the following formula (1) and a carbonic acid ester represented by the following formula (2): 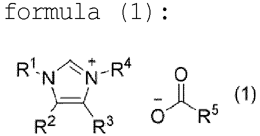 wherein R¹ and R⁴ are the same or different, and are each a C₁-C₂₀ hydrocarbon group optionally substituted with one or more heteroatoms; R² and R³ are the same or different, and are each a hydrogen atom or a C₁-C₂₀ hydrocarbon group optionally substituted with one or more heteroatoms; R² and R³, together with the carbon atoms to which they are attached, may form a ring structure; and R⁵ is a hydrogen atom or a C₁-C₂₀ hydrocarbon group optionally substituted with one or more heteroatoms; 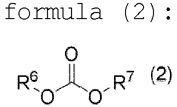 wherein R⁶ and R⁷ are the same or different, and are each a C₁-C₆ hydrocarbon group; and R⁶ and R⁷, together with the oxygen atoms to which they are attached, may form a ring structure; 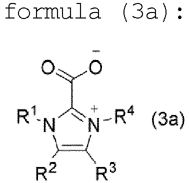 wherein R¹, R², R³, and R⁴ are as defined above.

2. The method for producing a carboxylate compound according to claim 1, wherein the carbonic acid ester represented by formula (2) is dimethyl carbonate.

3. The method for producing a carboxylate compound according to claim 1 or 2, wherein R² and R³ are hydrogen atoms.

4. A method for producing an amidate compound represented by the following formula (5), the method comprising the following steps 1 and 2:
step 1 of reacting an imidazolium carboxylic acid salt represented by formula (1) and a carbonic acid ester represented by formula (2) to obtain a reaction product (A): 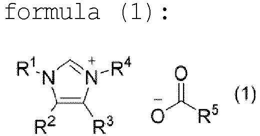 wherein R¹ and R⁴ are the same or different, and are each a C₁-C₂₀ hydrocarbon group optionally substituted with one or more heteroatoms; R² and R³ are the same or different, and are each a hydrogen atom or a C₁-C₂₀ hydrocarbon group optionally substituted with one or more heteroatoms; R² and R³, together with the carbon atoms to which they are attached, may form a ring structure; and R⁵ is a hydrogen atom or a C₁-C₂₀ hydrocarbon group optionally substituted with one or more heteroatoms; 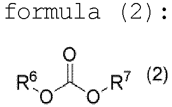 wherein R⁶ and R⁷ are the same or different, and are each a C₁-C₆ hydrocarbon group; and R⁶ and R⁷, together with the oxygen atoms to which they are attached, may form a ring structure; and
step 2 of optionally heating the reaction product (A) obtained in step 1, and reacting the reaction product (A) with a nitrogen-containing compound represented by formula (4) optionally under heating, to obtain an amidate compound represented by formula (5): 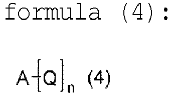 wherein A is a substituted or unsubstituted hydrocarbon group; Q is an -NCO group or -NHCO₂R⁸; R⁸ is a hydrocarbon group optionally substituted with one or more heteroatoms; and n is an integer of 1 or more; 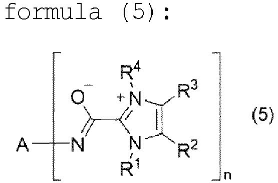 wherein A, R¹, R², R³, R⁴, and n are as defined above.

5. The method for producing an amidate compound according to claim 4, wherein the reaction product (A) comprises a carboxylate compound represented by formula (3a): 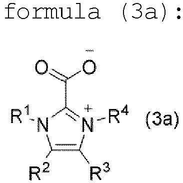 wherein R¹, R², R³, and R⁴ are as defined above.

6. The method for producing an amidate compound according to claim 4 or 5, wherein the nitrogen-containing compound represented by formula (4) is a nitrogen-containing compound represented by any of the following formulas (4-1) to (4-3):
formula (4-1): R⁹-Q (4-1)
wherein Q is as defined above; and R⁹ is a hydrocarbon group optionally substituted with one or more halogen atoms or a hydrocarbon group optionally substituted with one or more heteroatoms;
formula (4-2): Q-R¹⁰-Q (4-2)
wherein each Q is the same or different and is as defined above; R¹⁰ is a divalent hydrocarbon group optionally substituted with one or more halogen atoms or a divalent hydrocarbon group optionally substituted with one or more heteroatoms; 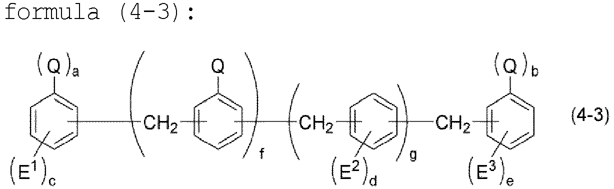 wherein each Q is the same or different and is as defined above; E¹, E², and E³ are each independently a hydrocarbon group optionally substituted with one or more halogen atoms, a hydrocarbon group optionally substituted with one or more heteroatoms, a halogen atom, a dialkylamino group, an alkoxy group, an aryloxy group, a nitro group, a cyano group, a sulfonyl group, an (alkylamino)carbonylamino group, a (dialkylamino)carbonylamino group, or an isocyanate group; f and g are each independently an integer of 0 to 4; a and b are each 0 or 1; and c, d, and e are each independently an integer of 0 to 4; provided that when f is 0, at least one of a or b is 1.

7. The method for producing an amidate compound according to any one of claims 4 to 6, wherein the carbonic acid ester represented by formula (2) is dimethyl carbonate.

8. The method for producing an amidate compound according to any one of claims 4 to 7, wherein R² and R³ are hydrogen atoms.

9. The method for producing an amidate compound according to any one of claims 6 to 8, wherein R⁹ is an aromatic hydrocarbon group optionally substituted with one or more halogen atoms or an aromatic hydrocarbon group optionally substituted with one or more heteroatoms; and R¹⁰ is a divalent aromatic hydrocarbon group optionally substituted with one or more halogen atoms or a divalent aromatic hydrocarbon group optionally substituted with one or more heteroatoms.
